# EUROPEAN PATENT SPECIFICATION

(11) **EP 3 438 125 B1**
(45) Date of publication and mention of the grant of the patent: **11.10.2023**
(21) Application number: 18186574.2
(22) Date of filing: 31.07.2018
(51) Int. Cl.: C07K 14/78, C12N 15/81, C12N 9/02

(54) **YEAST STRAINS AND METHODS FOR CONTROLLING HYDROXYLATION OF RECOMBINANT COLLAGEN**
HEFESTÄMME UND VERFAHREN ZUR STEUERUNG DER HYDROXYLIERUNG VON REKOMBINANTEM KOLLAGEN
SOUCHES DE LEVURE ET PROCÉDÉS DE CONTRÔLE DE L'HYDROXYLATION DE COLLAGÈNE RECOMBINANT

(30) Priority: 31.07.2017 US 201762539213 P
(43) Date of publication of application: 06.02.2019
(73) Proprietor: Modern Meadow, Inc., Nutley, New Jersey 07110 (US)
(72) Inventor: DAI, Lixin, LIVINGSTON, NJ 07039 (US); BORDEN, Julia, SAN MATEO, CA 94402 (US); NELSON, Jeffrey, NEW YORK, NY 11234 (US); RUEBLING-JASS, Kristin, UNION, NJ 07083 (US)
(74) Representative: Maiwald GmbH

(56) References cited:
- CA-A1- 2 134 994
- CN-A- 102 020 712
- JP-A- 2011 190 210
- US-A1- 2005 112 129
- US-A1- 2008 081 353
- US-A1- 2016 097 053
- "Put the proven strength of Pichia behind your protein expression", , XP002786968, Retrieved from the Internet: URL:https://assets.thermofisher.com/TFS-As sets/LSG/brochures/B-067202_Pichia_Flyer.p df [retrieved on 2018-11-28]
- Nokelainen M: "Recombinant human collagens: characterization of type ii collagen expressed in insect cells and production of types I-III collagen in the yeast pichia pastoris", Department of medical biochemistry. Faculty of medicine, University of Oulu Oulu University press, 2000, pages 1-72, XP002786969, Oulu Retrieved from the Internet: URL:http://jultika.oulu.fi/files/isbn95142 57383.pdf [retrieved on 2018-11-28]
- Invitrogen life technologies: , XP002786970, Retrieved from the Internet: URL:https://tools.thermofisher.com/content /sfs/vectors/ppicz_map.pdf [retrieved on 2018-11-28]
- ANNAMARI VUORELA ET AL: "Assembly of human prolyl 4-hydroxylase and type III collagen in the yeast Pichia pastoris: formation of a stable enzyme tetramer requires coexpression with collagen and assembly of a stable collagen requires coexpression with prolyl 4-hydroylase", EMBO (EUROPEAN MOLECULAR BIOLOGY ORGANIZATION) JOURNAL, WILEY, DE, vol. 16, no. 22, 1 January 1997 (1997-01-01), pages 6702-6712, XP002153732, ISSN: 0261-4189, DOI: 10.1093/EMBOJ/16.22.6702
- LIANG SHULI ET AL: "Identification and characterization of P-GCW14: a novel, strong constitutive promoter of Pichia pastoris", BIOTECHNOLOGY LETTERS, vol. 35, no. 11, November 2013 (2013-11), pages 1865-1871, XP002786971,

## Description

### BACKGROUND OF THE INVENTION

### Field of the Invention

This invention relates to a chimeric bovine collagen DNA sequence comprising a combination of a section of SEQ ID NO: 1 which has been optimized for expression in Pichia pastoris and a section of SEQ ID NO: 1 that is unoptimized, wherein the section of SEQ ID NO: 1 which has been optimized for expression in Pichia pastoris comprises 60 to 90% of the total length of the chimeric bovine collagen DNA sequence, and the section of SEQ ID NO: 1 that is unoptimized comprises 10 to 40% of the total length of the chimeric collagen DNA sequence, and wherein the section of SEQ ID NO: 1 which has been optimized for expression in Pichia pastoris encodes the amino acid sequence at the N-terminus of a bovine collagen molecule and the section of SEQ ID NO: 1 that is unoptimized encodes the amino acid sequence at the C-terminus of a bovine collagen molecule.

The present invention further relates to a strain of collagen-producing yeast comprising a vector, wherein the vector comprises the afore-mentioned chimeric bovine collagen DNA sequence.

The present invention also relates to a method for producing hydroxylated collagen comprising growing the afore-mentioned strain of collagen-producing yeast in a medium for 24 hours to 72 hours.

### Description of Related Art

Leather is used in a vast variety of applications, including for furniture upholstery, clothing, shoes, luggage, handbag and accessories, and automotive applications. The estimated global trade value in leather is approximately US $100 billion per year *(*Future Trends in the World Leather Products Industry and Trade, United Nations Industrial Development Organization, Vienna, 2010) and there is a continuing and increasing demand for leather products. New ways to meet this demand are required in view of the economic, environmental and social costs of producing leather. To keep up with technological and aesthetic trends, producers and users of leather products seek new materials exhibiting superior strength, uniformity, processability and fashionable and appealing aesthetic properties that incorporate natural components.

Given population growth and the global environment there will be a need for alternative materials that have leather-like aesthetics and improved functionalities. Leather is animal hide and consists almost entirely of collagen. There is a need for new sources of collagen that can be incorporated into biofabricated leather materials.

Production of biofabricated leather using recombinantly-expressed collagen faces a number of challenges including a need for a method for efficiently producing collagen in forms and quantities needed for diverse commercial applications. For some applications a softer and more permeable collagen component is desired; in others, a harder, more resistant and durable collagen component is needed.

Recombinant expression of some collagens and collagen-like proteins is known; see Bell, EP 1232182B1, *Bovine collagen and method for producing recombinant gelatin;* Olsen, et al., U.S. Patent No. 6,428,978, *Methods for the production of gelatin and full-length triple helical collagen in recombinant cells;* VanHeerde, et al., U.S. Patent No. 8,188,230, *Method for recombinant microorganism expression and isolation of collagen-like polypeptides.* Such recombinant collagens have not been used to produce leather or biofabricated leather products.

Vectors useful for expressing proteins in yeasts are known; see Ausubel et al., In: Current Protocols in Molecular Biology, Vol. 2, Chapter 13 Greene Publish. Assoc. & Wiley Interscience, 1988; Grant et al. (1987), Expression and Secretion Vectors for Yeast, in Methods in Enzymology, Ed. Wu & Grossman, Acad. Press, N.Y. 153:516-544; Glover (1986) DNA Cloning, Vol. II, IRL Press, Wash., D.C., Ch. 3; Bitter (1987), Heterologous Gene Expression in Yeast, in Methods in Enzymology, Eds. Berger & Kimmel, Acad. Press, N.Y. 152:673-684; and The Molecular Biology of the Yeast Saccharomyces, Eds. Strathern et al., Cold Spring Harbor Press, Vols. I and II (1982). Yeast expression vectors are commercially available, for example, as described in the catalogs at ThermoFisher Scientific (www._thermofisher.com); ATUM (https://www._atum.bio/products/expression-vectors/yeast); or IBA (https://www._iba-lifesciences.com/cloning-yeast-vectors.html)(each last accessed July 16, 2018).

*Pichia pastoris* is a yeast species that has been used to recombinantly express biotherapeutic proteins, such as human interferon gamma, see Razaghi, et al., Biologicals 45: 52-60 (2017). It has been used to express type III collagen and prolyl-4-hydroxylase, see Vuorela, et al., EMBO J. 16:6702-6712 (1997). Collagen and prolyl-4-hydroxylase have also been expressed in *Escherichia coli* to produce a collagenous material, see Pinkas, et al., ACS Chem. Biol. 6(4):320-324 (2011).

US 2008/081353 discloses methods, reagents (e.g. vectors) and host cells for the recombinant production of collagen, relating to the recombinant expression of a subunit of a collagen or procollagen and a collagen post-translational enzyme or subunit thereof.

CN 102 020 712 discloses a human-like collagen for a vaccine stabilizing agent and a production method thereof. The human-like collagen is obtained by fermenting yeast engineering bacteria which are named pichia pastoris X-33/col CGMCC NO.4187.

JP 2011 190210 discloses a transformant that produces a glycine repeat sequence protein and a method for obtaining a glycine repeat sequence protein.

Nokelainen M.: "Recombinant human collagens: characterization of type II collagen expressed in insect cells and production of types I-III collagen in the yeast pichia pastoris"; Department of medical biochemistry, Faculty of medicine, University of Oulu; Oulu University press, 2000, pages 1-72, discloses a production of a recombinant form of human type II collagen in insect cells. Furthermore, this thesis is concerned with the expression of recombinant human collagen in yeasts.

Annamari Vuorela et al.: "Assembly of human prolyl 4-hydroxylase and type III collagen in the yeast Pichia pastoris: formation of a stable enzyme tetramer requires coexpression with collagen and assembly of a stable collagen requires coexpression with prolyl-4-hydroxylase", EMBO (European Molecular Biology Organization) JOURNAL, WILEY, DE, vol. 16, No. 22, 1 January 1997, pages 6702-6712, is concerned with expression of prolyl 4-hydroxylase and concludes from the data that collagen synthesis in Pichia and probably also in other cells involves a highly unusual control mechanism.

CA 2 134 994 discloses a method of expressing a heterologous gene in mammalian cells and a recombinant DNA construct for use in this method.

Liang Shuli et al.: "Identification and characterization of P-GCW14: a novel, strong constitutive promoter of Pichia pastoris", BIOTECHNOLOGY LETTERS, vol. 35, no. 11, November 2013, pages 1865-1871, discloses the identification of a novel strong constitutive promoter in Pichia pastoris, P GCW14.

US 2016/097053 discloses isolated nucleic acids, expression methods, host cells, expression vectors, and DNA constructs for producing proteins, and proteins produced using the expression methods. More particularly, nucleic acids isolated from Pichia pastoris are disclosed therein, wherein the nucleic acids have promoter activity. Moreover, this patent document relates to expression methods, host cells, expression vectors, and DNA constructs, for using the Pichia pastoris promoters to produce proteins, and to the proteins produced using the expression methods.

US 2005/112129 discloses compositions of matter useful for the diagnosis and treatment of tumor in mammals and to methods of using those compositions of matter for the same.

The use of codon-modification to provide tropocollagen with a select degree of hydroxylation, thus providing a range of different collagen materials for use in production of bioengineered leathers, has not been previously explored.

The inventors sought to address these challenges by engineering recombinant yeasts which can abundantly express collagen in different forms characterized by a selective degree of hydroxylation.

### SUMMARY OF THE INVENTION

One aspect of the invention is directed to a chimeric bovine collagen DNA sequence as claimed in claim 1.

Another aspect of the invention is directed to a strain of collagen-producing yeast as claimed in claim 4.

Yet another aspect of the invention is directed to a method for producing hydroxylated collagen as claimed in claim 6.

### BRIEF DESCRIPTION OF THE FIGURES

FIG. 1 shows the vector diagram of MMV-63 which was designed to produce non-hydroxylated collagen.
FIG. 2 shows the vector diagram of MMV-77 which was designed to produce non-hydroxylated collagen.
FIG. 3 shows the vector diagram of MMV-129 which was designed to produce non-hydroxylated collagen.
FIG. 4 shows the vector diagram of MMV-130 which was designed to produce non-hydroxylated collagen.
FIG. 5 shows the vector diagram of MMV-78 which was designed to produce hydroxylated collagen.
FIG. 6 shows the vector diagram of MMV-94 which was designed to produce hydroxylated collagen.
FIG. 7 shows the vector diagram of MMV-156 which was designed to produce hydroxylated collagen.
FIG. 8 shows the vector diagram of MMV-191 which was designed to produce hydroxylated collagen.
FIG. 9 shows an all-in-one vector MMV-208 which was designed to produce non-hydroxylated or hydroxylated collagen.
FIG. 10 shows the vector diagram of MMV-84.
FIG. 11 shows the vector diagram of MMV-150.
FIG. 12 shows the vector diagram of MMV-140.
FIG. 13 shows the vector diagram of MMV-132.
FIG. 14 shows the vector diagram of MMV-193.
FIG. 15 shows the vector diagram of MMV-194
FIG. 16 shows the vector diagram of MMV-195,
FIG. 17 shows the vector diagram of MMV-197.
FIG. 18 shows the vector diagram of MMV-198.
FIG. 19 shows the vector diagram of MMV-199.
FIG. 20 shows the vector diagram of MMV-200.
FIG. 21 shows the vector diagram of MMV-128.

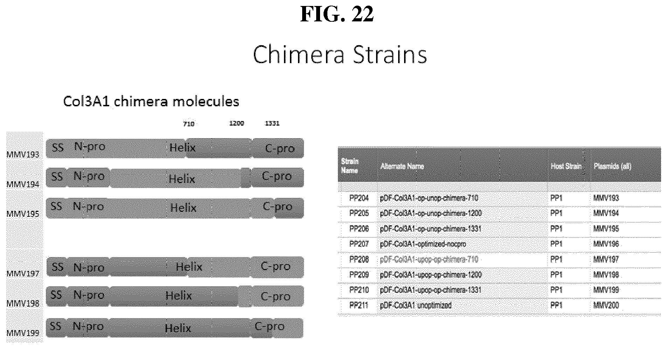

FIG. 22 describes Col3A1 chimera molecules.

### DETAILED DESCRIPTION OF THE INVENTION AND OF DISCLOSURE

Subject matter referred to herein in the following as "disclosure" or "embodiment of the disclosure" is not according to the invention and is present for illustration purposes only.

As exemplified herein, *Pichia pastoris* was used to express recombinant Type III bovine collagen with different degrees of hydroxylation. Hydroxylation of recombinant collagen was accomplished by co-expression of bovine P4HA and bovine P4HB which respectively encode the alpha and beta subunits bovine prolyl-4-hydroxylase. However, the disclosure is not limited to products and expression of Type III collagen and may be practiced with polynucleotides encoding the subunits of other kinds of collagens as well as with enzymes that hydroxylate proline residues, lysine residues, or both proline and lysine residues. Type III tropocollagen is a homotrimer. However, in some embodiments a collagen will form a heterotrimer composed of different polypeptide chains, such as Type I collagen which is initially composed of two pro-α1(I) chains and one pro- α2(I) chain.

*Collagen.* Collagen is the main component of leather. Skin, or animal hide, contains significant amounts of collagen, a fibrous protein. Collagen is a generic term for a family of at least 28 distinct collagen types; animal skin is typically Type I collagen, although other types of collagen can be used in forming leather including type III collagen. The term "collagen" encompasses unprocessed *(e.g*., procollagens) as well as post-translationally modified and proteolysed collagens having a triple helical structure.

Collagens are characterized by a repeating triplet of amino acids, -(Gly-X-Y)n-, and approximately one-third of the amino acid residues in collagen are glycine. X is often proline and Y is often hydroxyproline, though there may be up to 400 possible Gly-X-Y triplets. Different animals may produce collagens having different amino acid compositions, which can impart different properties on the collagen and produce leathers having different properties or appearances.

The structure of collagen can consist of three intertwined peptide chains of differing lengths. Collagen triple helices (or monomers) may be produced from alpha-chains of about 1,050 amino acids long, so that the triple helix takes the form of a rod of about approximately 300 nm long, with a diameter of approximately 1.5 nm.

Collagen fibers may have a range of diameters depending on the type of animal hide. In addition to type I collagen, skin (hides) may include other types of collagen as well, including type III collagen (reticulin), type IV collagen, and type VII collagen.

Various types of collagen exist throughout the mammalian body. For example, besides being the main component of skin and animal hide, Type I collagen also exists in cartilage, tendon, vascular ligature, organs, muscle, and the organic portion of bone. Successful efforts have been made to isolate collagen from various regions of the mammalian body in addition to the animal skin or hide. Decades ago, researchers found that at neutral pH, acid-solubilized collagen self-assembled into fibrils composed of the same cross-striated patterns observed in native tissue; Schmitt F.O. J. Cell. Comp Physiol. 1942;20:11. This led to use of collagen in tissue engineering and a variety of biomedical applications. In more recent years, collagen has been harvested from bacteria and yeast using recombinant techniques.

Collagens are formed and stabilized through a combination of physical and chemical interactions including electrostatic interactions such as salt bridging, hydrogen bonding, Van der Waals interactions, dipole-dipole forces, polarization forces, hydrophobic interactions, and covalent bonding often catalyzed by enzymatic reactions. Various distinct collagen types have been identified in vertebrates including bovine, ovine, porcine, chicken, and human collagens.

The disclosure may be practiced with polynucleotides encoding one or more types of collagen. Generally, the collagen types are numbered by Roman numerals and the chains found in each collagen type are identified by Arabic numerals. Detailed descriptions of structure and biological functions of the various different types of naturally occurring collagens are available in the art; see, e.g., Ayad et al. (1998) The Extracellular Matrix Facts Book, Academic Press, San Diego, CA; Burgeson, R E., and Nimmi (1992) "Collagen types: Molecular Structure and Tissue Distribution" in Clin. Orthop. 282:250-272; Kielty, C. M. et al. (1993) "The Collagen Family: Structure, Assembly And Organization In The Extracellular Matrix," Connective Tissue And Its Heritable Disorders, Molecular Genetics, And Medical Aspects, Royce, P. M. and B. Steinmann eds., Wiley-Liss, NY, pp. 103-147; and Prockop, D.J- and K.I. Kivirikko (1995) "Collagens: Molecular Biology, Diseases, and Potentials for Therapy," Annu. Rev. Biochem., 64:403-434.)

Type I collagen is the major fibrillar collagen of bone and skin comprising approximately 80-90% of an organism's total collagen. Type I collagen is the major structural macromolecule present in the extracellular matrix of multicellular organisms and comprises approximately 20% of total protein mass. Type I collagen is a heterotrimeric molecule comprising two α1(I) chains and one α2(I) chain, encoded by the COL1A1 and COL1A2 genes, respectively. *In vivo,* assembly of Type I collagen fibrils, fibers, and fiber bundles takes place during development and provides mechanical support to the tissue while allowing for cellular motility and nutrient transport. Other collagen types are less abundant than type I collagen and exhibit different distribution patterns. For example, type II collagen is the predominant collagen in cartilage and vitreous humor, while type III collagen is found at high levels in blood vessels and to a lesser extent in skin.

Type II collagen is a homotrimeric collagen comprising three identical al(II) chains encoded by the COL2A1 gene. Purified type II collagen may be prepared from tissues by, methods known in the art, for example, by procedures described in Miller and Rhodes (1982) Methods In Enzymology 82:33-64.

Type III collagen is a major fibrillar collagen found in skin and vascular tissues. Type III collagen is a homotrimeric collagen comprising three identical α1(III) chains encoded by the COL3A1 gene. Methods for purifying type III collagen from tissues can be found in, for example, Byers et al. (1974) Biochemistry 13:5243-5248; and Miller and Rhodes, *supra* and may be used in conjunction with collagen expressed by a method of the invention according to claim 6.

Type IV collagen is found in basement membranes in the form of sheets rather than fibrils. Most commonly, type IV collagen contains two α1(IV) chains and one α2(IV) chain. The particular chains comprising type IV collagen are tissue-specific. Type IV collagen may be purified using, for example, the procedures described in Furuto and Miller (1987) Methods in Enzymology, 144:41-61, Academic Press.

Type V collagen is a fibrillar collagen found in, primarily, bones, tendon, cornea, skin, and blood vessels. Type V collagen exists in both homotrimeric and heterotrimeric forms. One form of type V collagen is a heterotrimer of two α1(V) chains and one α2(V) chain. Another form of type V collagen is a heterotrimer of α1(V), α2(V), and α3(V) chains. A further form of type V collagen is a homotrimer of α1(V). Methods for isolating type V collagen from natural sources can be found, for example, in Elstow and Weiss (1983) Collagen Rel. Res. 3:181-193, and Abedin et al. (1982) Biosci. Rep. 2:493-502.

Type VI collagen has a small triple helical region and two large non-collagenous remainder portions. Type VI collagen is a heterotrimer comprising α1(VI), α2(VI), and α3(VI) chains. Type VI collagen is found in many connective tissues. Descriptions of how to purify type VI collagen from natural sources can be found, for example, in Wu et al. (1987) Biochem. J. 248:373-381, and Kielty et al. (1991) J. Cell Sci. 99:797-807.

Type VII collagen is a fibrillar collagen found in particular epithelial tissues. Type VII collagen is a homotrimeric molecule of three α1(VII) chains. Descriptions of how to purify type VII collagen from tissue can be found in, for example, Lunstrum et al. (1986) J. Biol. Chem. 261:9042-9048, and Bentz et al. (1983) Proc. Natl. Acad. Sci. USA 80:3168-3172. Type VIII collagen can be found in Descemet's membrane in the cornea. Type VIII collagen is a heterotrimer comprising two α1(VIII) chains and one α2(VIII) chain, although other chain compositions have been reported. Methods for the purification of type VIII collagen from nature can be found, for example, in Benya and Padilla (1986) J. Biol. Chem. 261:4160-4169, and Kapoor et al. (1986) Biochemistry 25:3930-3937.

Type IX collagen is a fibril-associated collagen found in cartilage and vitreous humor. Type IX collagen is a heterotrimeric molecule comprising α1(IX), α2(IX), and α3(IX) chains. Type IX collagen has been classified as a FACIT (Fibril Associated Collagens with Interrupted Triple Helices) collagen, possessing several triple helical domains separated by non-triple helical domains. Procedures for purifying type IX collagen can be found, for example, in Duance, et al. (1984) Biochem. J. 221:885-889; Ayad et al. (1989) Biochem. J. 262:753-761; and Grant et al. (1988) The Control of Tissue Damage, Glauert, A. M., ed., Elsevier Science Publishers, Amsterdam, pp. 3-28.

Type X collagen is a homotrimeric compound of α1(X) chains. Type X collagen has been isolated from, for example, hypertrophic cartilage found in growth plates; see, e.g., Apte et al. (1992) Eur J Biochem 206 (1):217-24.

Type XI collagen can be found in cartilaginous tissues associated with type II and type IX collagens, and in other locations in the body. Type XI collagen is a heterotrimeric molecule comprising α1(XI), α2(XI), and α3(XI) chains. Methods for purifying type XI collagen can be found, for example, in Grant et al., *supra.*

Type XII collagen is a FACIT collagen found primarily in association with type I collagen. Type XII collagen is a homotrimeric molecule comprising three α1(XII) chains. Methods for purifying type XII collagen and variants thereof can be found, for example, in Dublet et al. (1989) J. Biol. Chem. 264:13150-13156; Lunstrum et al. (1992) J. Biol. Chem. 267:20087-20092; and Watt et al. (1992) J. Biol. Chem. 267:20093-20099.

Type XIII is a non-fibrillar collagen found, for example, in skin, intestine, bone, cartilage, and striated muscle. A detailed description of type XIII collagen may be found, for example, in Juvonen et al. (1992) J. Biol. Chem. 267: 24700-24707.

Type XIV is a FACIT collagen characterized as a homotrimeric molecule comprising α1(XIV) chains. Methods for isolating type XIV collagen can be found, for example, in Aubert-Foucher et al. (1992) J. Biol. Chem. 267:15759-15764, and Watt et al., *supra.*

Type XV collagen is homologous in structure to type XVIII collagen. Information about the structure and isolation of natural type XV collagen can be found, for example, in Myers et al. (1992) Proc. Natl. Acad. Sci. USA 89:10144-10148; Huebner et al. (1992) Genomics 14:220-224; Kivirikko et al. (1994) J. Biol. Chem. 269:4773-4779; and Muragaki, J. (1994) Biol. Chem. 264:4042-4046.

Type XVI collagen is a fibril-associated collagen, found, for example, in skin, lung fibroblast, and keratinocytes. Information on the structure of type XVI collagen and the gene encoding type XVI collagen can be found, for example, in Pan et al. (1992) Proc. Natl. Acad. Sci. USA 89:6565-6569; and Yamaguchi et al. (1992) J. Biochem. 112:856-863.

Type XVII collagen is a hemidesmosal transmembrane collagen, also known at the bullous pemphigoid antigen. Information on the structure of type XVII collagen and the gene encoding type XVII collagen can be found, for example, in Li et al. (1993) J. Biol. Chem. 268(12):8825-8834; and McGrath et al. (1995) Nat. Genet. 11(1):83-86.

Type XVIII collagen is similar in structure to type XV collagen and can be isolated from the liver. Descriptions of the structures and isolation of type XVIII collagen from natural sources can be found, for example, in Rehn and Pihlajaniemi (1994) Proc. Natl. Acad. Sci USA 91:4234- 4238; Oh et al. (1994) Proc. Natl. Acad. Sci USA 91:4229-4233; Rehn et al. (1994) J. Biol. Chem. 269:13924-13935; and Oh et al. (1994) Genomics 19:494-499.

Type XIX collagen is believed to be another member of the FACIT collagen family, and has been found in mRNA isolated from rhabdomyosarcoma cells. Descriptions of the structures and isolation of type XIX collagen can be found, for example, in Inoguchi et al. (1995) J. Biochem. 117:137-146; Yoshioka et al. (1992) Genomics 13:884-886; and Myers et al., J. Biol. Chem. 289:18549-18557 (1994).

Type XX collagen is a newly found member of the FACIT collagenous family, and has been identified in chick cornea; see, e.g., Gordon et al. (1999) FASEB Journal 13:A1119; and Gordon et al. (1998), IOVS 39:S1128.

One or more kinds of collagen may be expressed using a method of the disclosure and the expressed collagen further processed or purified as described by the references cited above.

The term "collagen" refers to any one of the known collagen types, including collagen types I through XX described above, as well as to any other collagens, whether natural, synthetic, semi-synthetic, or recombinant. It includes all of the collagens, modified collagens and collagen-like proteins described herein. The term also encompasses procollagens and collagen-like proteins or collagenous proteins comprising the motif (Gly-X-Y)n where n is an integer. It encompasses molecules of collagen and collagen-like proteins, trimers of collagen molecules, fibrils of collagen, and fibers of collagen fibrils. It also refers to chemically, enzymatically or recombinantly-modified collagens or collagen-like molecules that can be fibrillated as well as fragments of collagen, collagen-like molecules and collagenous molecules capable of assembling into a nanofiber. Recombinant collagen molecules whether native or engineered will generally comprise the repeated -(Gly-X-Y)n- sequence described herein.

*Hydroxylation of proline and lysine residues in collagen.* The principal post-translational modifications of the polypeptides of collagen are the hydroxylation of proline and/or lysine residues to yield 4-hydroxyproline, 3-hydroxyproline (Hyp) and/or hydroxylysine (Hyl), and glycosylation of the hydroxylysyl residues. These modifications are catalyzed by three hydroxylases--prolyl 4-hydroxylase, prolyl 3-hydroxylase, and lysyl hydroxylase-- and two glycosyl transferases. *In vivo* these reactions occur until the polypeptides form the triple-helical collagen structure, which inhibits further modifications.

*Prolyl-4-hydroxylase.* This enzyme catalyzes hydroxylation of proline residues to (2S,4R)-4-hydroxyproline (Hyp). Gorres, et al., Critical Reviews in Biochemistry and Molecular Biology 45 (2): (2010). The Examples below employ tetrameric bovine prolyl-4-hydroxylase (2 alpha and 2 beta chains) encoded by P4HA (SEQ ID NO: 54) and P4HB (SEQ ID NO: 52), however, isoforms, orthologs, variants, fragments and prolyl-4-hydroxylase from non-bovine sources may also be used as long as they retain hydroxylase activity in a yeast host cell. P4HA1 is further described by http://_www.omim.org/entry/176710 and P4HB1 and P4HB1 by http://www.omim.org/entry/176790 .

*Prolyl 3-hydroxylase.* This enzyme catalyzes hydroxylation of proline residues. Prolyl 3-hydroxylase 1 precursor *[Bos tourus]* is described by NCBI Reference Sequence: NP_001096761.1 or by NM_001103291.1 (SEQ ID NO: 48). For further description see Vranka, et al., J. Biol. Chem. 279: 23615-23621 (2004) or hhttp://_www.omim.org/entry/610339 (last accessed July 14, 2017). This enzyme may be used in its native form. However, isoforms, orthologs, variants, fragments and prolyl-3-hydroxylase from non-bovine sources may also be used as long as they retain hydroxylase activity in a yeast host cell.

*Lysyl hydroxylase.* Lysyl hydroxylase (EC 1.14.11.4) catalyzes the formation of hydroxylysine in collagens and other proteins with collagen-like amino acid sequences, by the hydroxylation of lysine residues in X-lys-gly sequences. The enzyme is a homodimer consisting of subunits with a molecular mass of about 85 kD. No significant homology has been found between the primary structures of lysyl hydroxylase and the 2 types of subunits of prolyl-4-hydroxylase (176710, 176790) despite the marked similarities in kinetic properties between these 2 collagen hydroxylases. The hydroxylysine residues formed in the lysyl hydroxylase reaction have 2 important functions: first, their hydroxy groups serve as sites of attachment for carbohydrate units, either the monosaccharide galactose or the disaccharide glucosylgalactose; and second, they stabilize intermolecular collagen crosslinks.

PLOD1 procollagen-lysine,2-oxoglutarate 5-dioxygenase 1 [ *Bos taurus* (cattle) ] is described by Gene ID: 281409, updated on 25-May-2017 in https://www.ncbi.nlm.nih.gov/gene/281409 (last accessed July 14, 2017). Another example is described by SEQ ID NO: 50 which describes *Bos taurus* lysyl oxidase (LOX). This enzyme may be used in its native form. However, isoforms, orthologs, variants, fragments and lysyl hydroxylase from non-bovine sources may also be used as long as they retain hydroxylase activity in a yeast host cell.

*Assay of degree of hydroxylation of proline residues in recombinant collagen.* The degree of hydroxylation of proline residues in recombinant collagen may be assayed by known methods, including by liquid chromatography-mass spectrometry as described by Chan, et al., BMC Biotechnology 12:51 (2012).

**Assay of degree of hydroxylation of lysine residues in recombinant collagen.** Lysine Hydroxylation and cross-linking of collagen is described by Yamauchi, et al., Methods in Molecular Biology, vol. 446, pages 95-108.; Humana Press (2008). The degree of hydroxylation of lysine residues in recombinant collagen may be assayed by known methods, including by the method described by Hausmann, Biochimica et Biophysica Acta (BBA) - Protein Structure 133(3): 591-593 (1967).

*Collagen Melting Point.* The degree of hydroxylation of proline, lysine or proline and lysine residues in collagen may be estimated by melting temperature of a hydrated collagen, such as a hydrogel compared to a control collagen having a known content of hydroxylated amino acid residues. Collagen melting temperatures can range from 25-40°C with more highly hydroxylated collagens generally having higher melting temperatures. This range includes all intermediate subranges and values including 25, 26, 28, 29, 30, 31, 32, 33, 34, 35, 36, 37, 38, 39 and 40.

*Codon-modification.* This process includes alteration of a polynucleotide sequence encoding collagen, such as collagen DNA sequence found in nature, to modify the amount of recombinant collagen expressed by a yeast, such as *Pichia pastoris,* to modify the amount of recombinant collagen secreted by the recombinant yeast, to modify the speed of expression of recombinant collagen in the recombinant yeast, or to modify the degree of hydroxylation of lysine or proline residues in the recombinant collagen. Codon modification may also be applied to other proteins such as hydroxylases for similar purposes or to target hydroxylases to particular intracellular or extracellular compartments, for example to target a proline hydroxylase to the same compartment, such as the endoplasmic reticulum, as recombinant collagen molecule.

Codon selections may be made based on effect on RNA secondary structure, effect on transcription and gene expression, effect on the speed of translation elongation, and/or the effect on protein folding.

Codons encoding collagen or a hydroxylase may be modified to reduce or increase secondary structure in mRNA encoding recombinant collagen or the hydroxylase or may be modified to replace a redundant codon with a codon which, on average, is used most frequently by a yeast host cell based on all the protein-coding sequences in the yeast (e.g., codon sampling), is used least frequently by a yeast host cell based on all the protein-coding sequences in the yeast (e.g., codon sampling), or redundant codons that appear in proteins that are abundantly-expressed by yeast host cells or which appear in proteins that are secreted by yeast host cells *(e.g*., a codon selection based on a High Codon Adaptation Index that makes the gene "look like" a highly expressed gene or gene encoding a secretable protein from the expression host).

Codon-modification may be applied to all or part of a protein-coding sequence, for example, to at least one of the first, second, third, fourth, fifth, sixth, seventh, eighth, ninth or tenth 10% of a coding-sequence or combinations thereof. It may also be applied selectively to a codon encoding a particular amino acid or to codons encoding some but not all amino acids that are encoded by redundant codons. For example, only codons for leucine and phenylalanine may be codon-modified as described above. Amino acids encoded by more than one codon are described by the codon table at which is well-known in the art in https://en.wikipedia.org/wiki/DNA_codon_table (last accessed July 13, 2017).

Codon-modification includes the so-called codon-optimization methods described by https://www.atum.bio/services/genegps (last accessed July 13, 2017), by https://www.idtdna.com/CodonOpt; by https://www.ncbi.nlm.nih.gov/pmc/articles/PMC1523223/, or by https://en.wikipedia.org/wiki/DNA2.0Algorithm.

Codon-modification also includes selection of codons so as to permit formation of mRNA secondary structure or to minimize or eliminate secondary structure. An example of this is making codon selections so as to eliminate, reduce or weaken secondary structure strong secondary structure at or around a ribosome-binding site or initiation codon.

*Collagen Fragments.* A recombinant collagen molecule can comprise a fragment of the amino acid sequence of a native collagen molecule capable of forming tropocollagen (trimeric collagen) or a modified collagen molecule or truncated collagen molecule having an amino acid sequence at least 70, 80, 90, 95, 96, 97, 98, or 99% identical or similar to a native collagen amino acid sequence (or to a fibril forming region thereof or to a segment substantially comprising [Gly-X-Y]n), such as those of amino acid sequences of Col1A1, Col1A2, and Col3A1, described by Accession Nos. NP_001029211.1 (https://_www.ncbi.nlm.nih.gov/protein/774042S2, last accessed February 9, 2017), NP_776945.1 (https://_www.ncbi.nlm.nih.gov/protein/278062S7 last accessed February 9, 2017) and NP_001070299.1 (https://_www.ncbi.nlm.nih.gov/protein/116003881 last accessed February 9, 2017).

A gene encoding collagen or a hydroxylase may be truncated or otherwise modified to add or remove sequences. Such modifications may be made to customize the size of a polynucleotide or vector, to target the expressed protein to the endoplasmic reticulum or other cellular or extracellular compartment, or to control the length of an encoded protein. For example, the inventors found that constructs containing only the Pre sequence often work better than those containing the entire Pre-pro sequence. The Pre sequence was fused to P4HB to localize P4HB in the ER where collagen localizes as well.

Modified coding sequences for collagens and hydroxylases. A polynucleotide coding sequence for collagen or a hydroxylase, or other proteins, may be modified to encode a protein that is at least 70, 80, 90, 95, 96, 97, 98, or 100% identical or similar to a known amino acid sequence and which retains the essential properties of the unmodified molecule, for example, the ability to form tropocollagen or the ability to hydroxylase proline or lysine residues in collagen. Glycosylation sites in a collagen molecule may be removed or added. Modifications may be made to facilitate collagen yield or its secretion by a yeast host cell or to change its structural, functional, or aesthetic properties. A modified collagen or hydroxylase coding sequence may also be codon-modified as described herein.

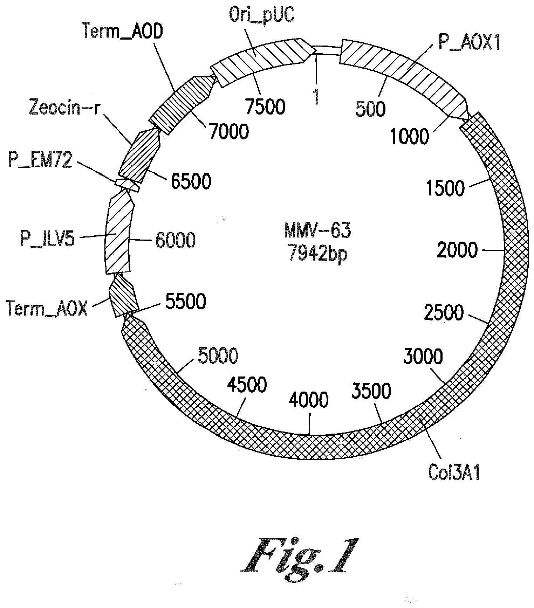

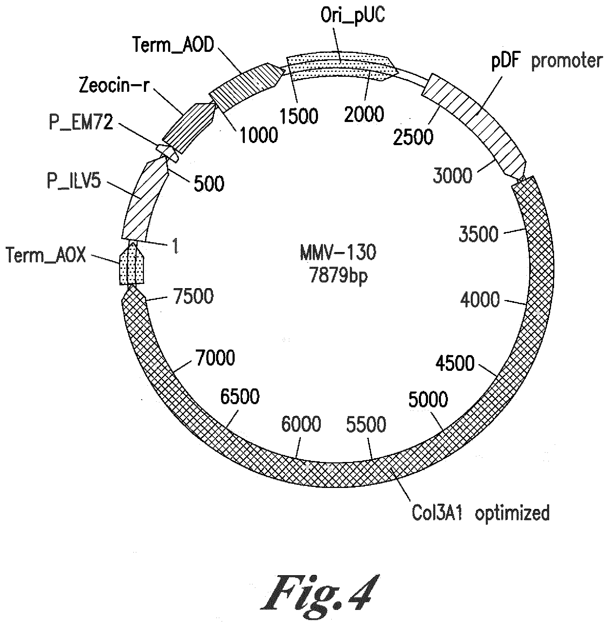

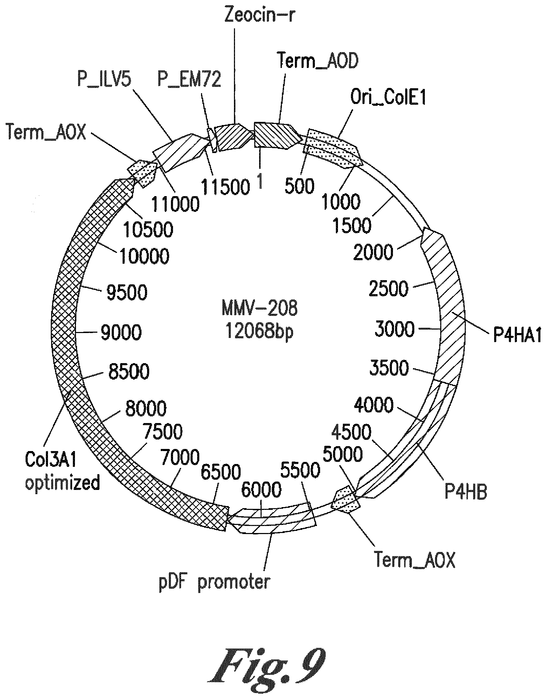

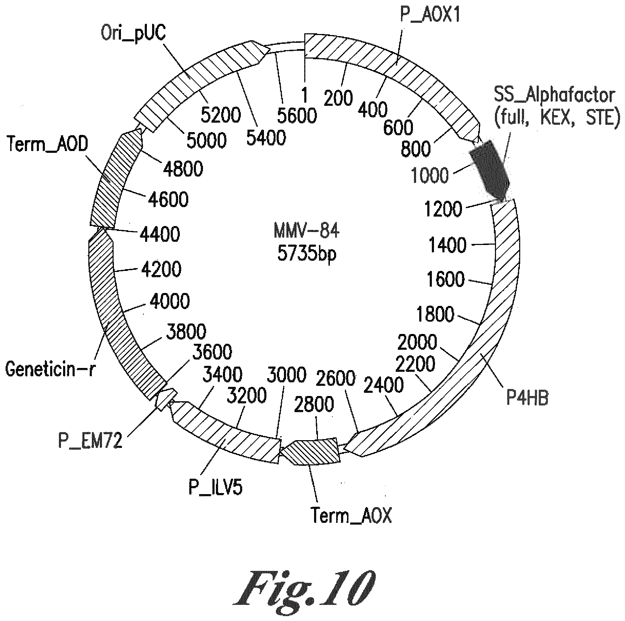

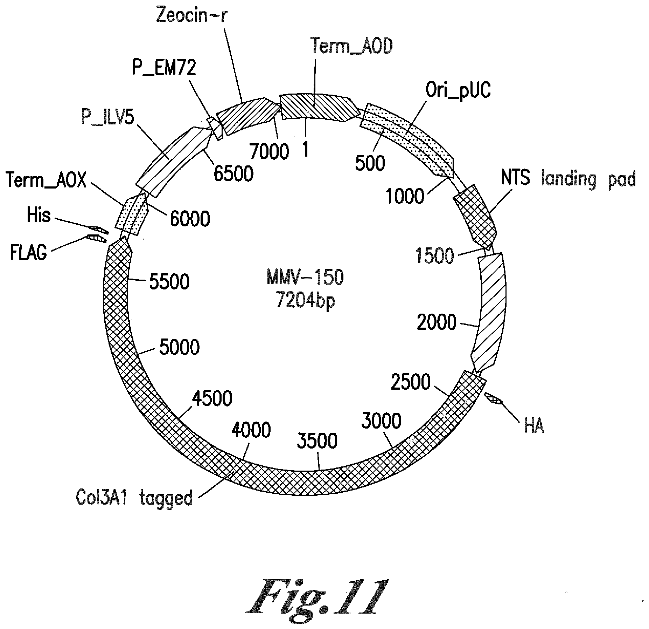

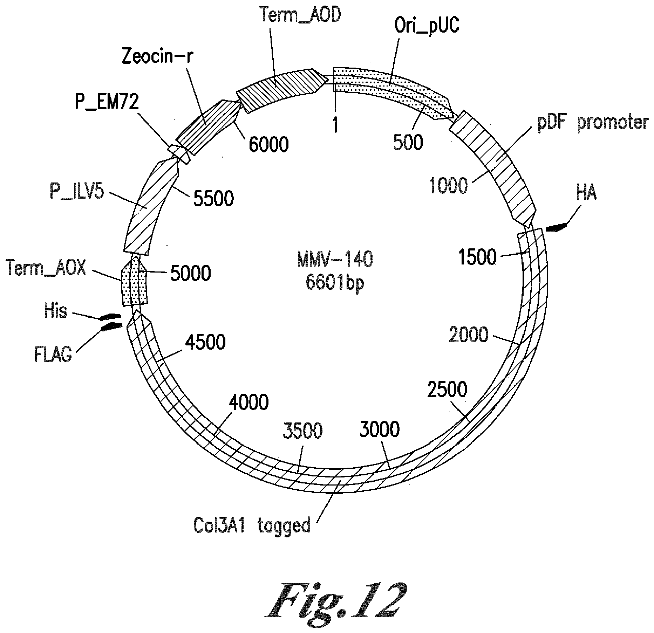

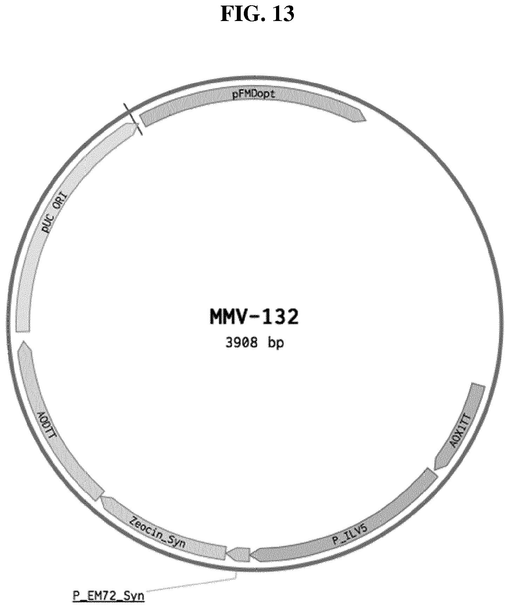

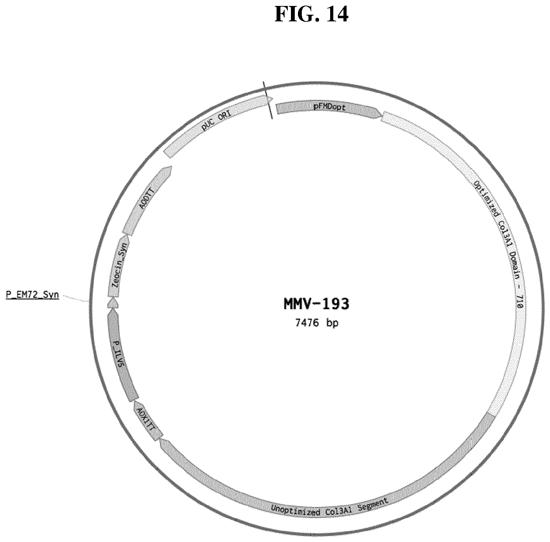

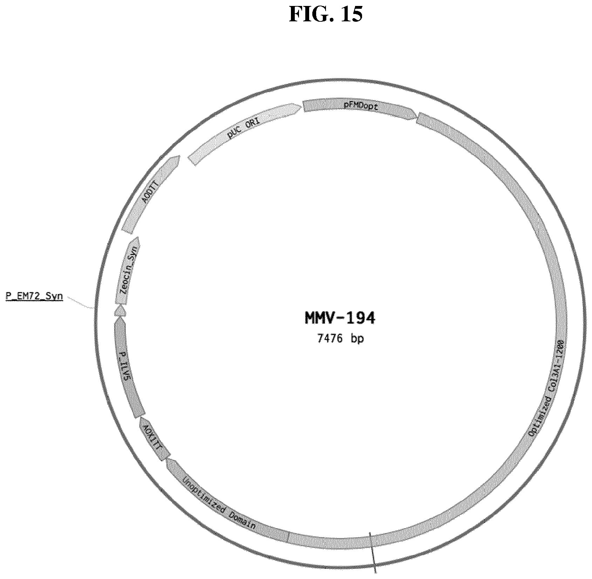

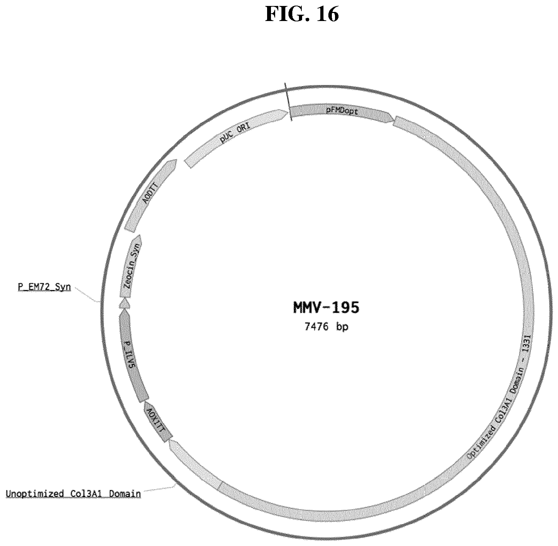

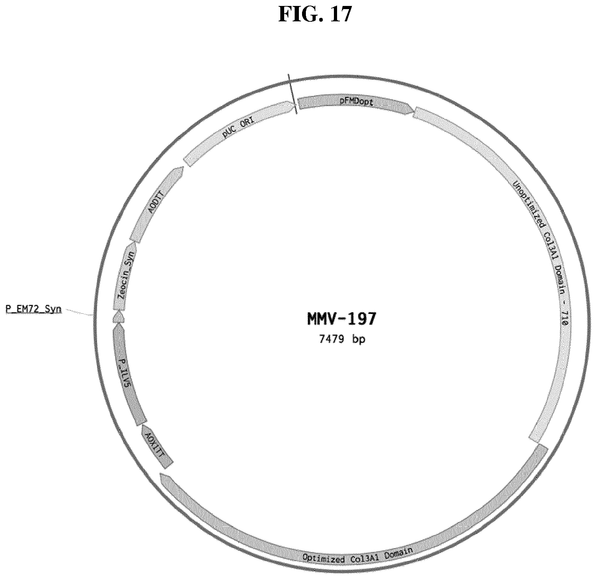

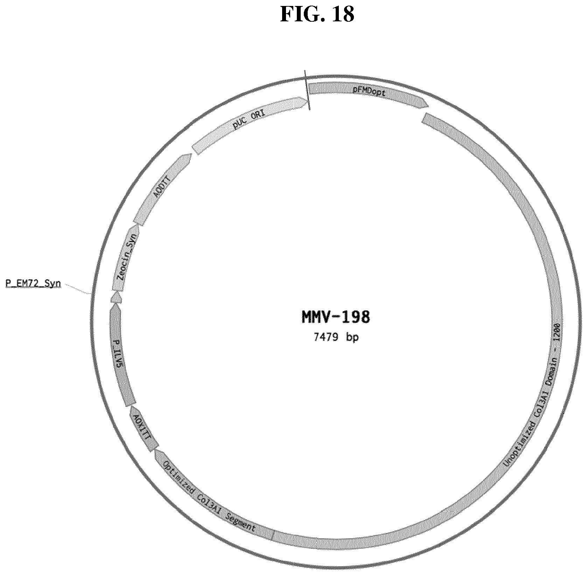

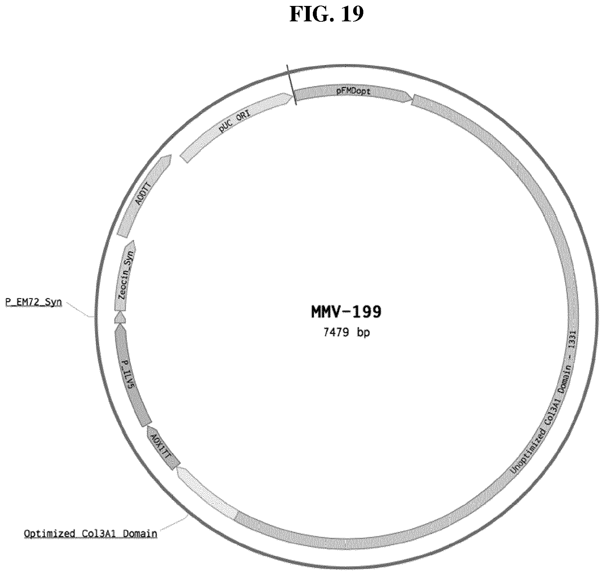

The terms "native collagen", "native polypeptide" or "native polynucleotide" refer to polypeptide or polynucleotide sequence as they are found in nature, for example, without deletion, addition of substitution of amino acid residues or for, polynucleotides, without alteration of the native sequence, for example, by deletion, insertion or substitution of a nucleotide, such as alteration by codon-modification. The types of collagens and enzymes described herein include their native forms as well as modified forms that retain a biological activity of the native collagen or enzyme. Modified forms of polynucleotides and polypeptides may be identified by those having a particular degree of sequence identity or similarity to a corresponding native sequence. Modified polynucleotide sequences also include those having 70, 80, 90, 95, 96, 97, 98, 99 or 100% sequence identity or similarity to any of the vectors described herein or to any of the polynucleotide elements that make up these vectors as depicted for example in FIGS. 1-20.

BLASTN may be used to identify a polynucleotide sequence having at least 70%, 75%, 80%, 85%, 87.5%, 90%, 92.5%, 95%, 97.5%, 98%, 99% or <100% sequence identity to a reference polynucleotide such as a polynucleotide encoding a collagen, one or more hydroxylases described herein, or signal, leader or secretion peptides or any other proteins disclosed herein. A representative BLASTN setting modified to find highly similar sequences uses an Expect Threshold of 10 and a Wordsize of 28, max matches in query range of 0, match/mismatch scores of 1/-2, and linear gap cost. Low complexity regions may be filtered or masked. Default settings of a Standard Nucleotide BLAST are described in https://_blast.ncbi.nlm.nih.gov/Blast.cgi?PROGRAM=blastn&PAGE TYPE=BIastSearch&LINK _LOC=blasthome (last accessed July 13, 2017).

BLASTP can be used to identify an amino acid sequence having at least 70%, 75%, 80%, 85%, 87.5%, 90%, 92.5%, 95%, 97.5%, 98%, 99% or <100% sequence identity, or similarity to a reference amino acid, such as a collagen amino acid sequence, using a similarity matrix such as BLOSUM45, BLOSUM62 or BLOSUM80 where BLOSUM45 can be used for closely related sequences, BLOSUM62 for midrange sequences, and BLOSUM80 for more distantly related sequences. Unless otherwise indicated a similarity score will be based on use of BLOSUM62. When BLASTP is used, the percent similarity is based on the BLASTP positives score and the percent sequence identity is based on the BLASTP identities score. BLASTP "Identities" shows the number and fraction of total residues in the high scoring sequence pairs which are identical; and BLASTP "Positives" shows the number and fraction of residues for which the alignment scores have positive values and which are similar to each other. Amino acid sequences having these degrees of identity or similarity or any intermediate degree of identity or similarity to the amino acid sequences disclosed herein are contemplated and encompassed by this disclosure. A representative BLASTP setting that uses an Expect Threshold of 10, a Word Size of 3, BLOSUM 62 as a matrix, and Gap Penalty of 11 (Existence) and 1 (Extension) and a conditional compositional score matrix adjustment. Other default settings for BLASTP are described in: https://blast.ncbi.nlm.nih.gov/Blast.cgi?PROGRAM=blastp&PAGE_TYPE=BlastSearch&LINK_ LOC=blasthome (last accessed July 13, 2017).

The term "derivative thereof", "modified sequence" or "analog" as applied to the polypeptides disclosed herein, refers to a polypeptide comprising an amino acid sequence that is at least 70, 80, 90, 95, or 99% identical or similar to the amino acid sequence of a biologically active molecule. In some embodiments, the derivative comprises an amino acid sequence that is at least 75%, 80%, 85%, 86%, 87%, 88%, 89%, 90%, 91%, 92%, 93%, 94%, 95%, 96%, 97%, 98%, or 99% identical to the amino acid sequence of a native or previously engineered sequence. The derivative may comprise additions, deletions, substitutions, or a combination thereof to the amino acid sequence of a native or previously engineered molecule. For example, a derivative may incorporate or delete 1, 2, 3, 4, 5, 6, 7, 8, 9, 10 or more proline or lysine residues compared to a native collagen sequence. Such selections may be made to modify the looseness or tightness of a recombinant tropocollagen or fibrillated collagen.

A derivative may include a mutant polypeptide with 1, 2, 3, 4, 5, 6, 7, 8, 9, 10, 11-15, 16-20, 21-25, or 26-30 additions, substitutions, or deletions of amino acid residues. Additions or substitutions also include the use of non-naturally occurring amino acids or modified amino acids. A derivative may also include chemical modifications to a polypeptide, such as crosslinks between cysteine residues, or hydroxylated or glycosylated residues. Derivatives include those of all polypeptides, including collagens and enzymes, disclosed herein. Generally, a derivative will have at least one biological activity of the unmodified parent molecule, thus an enzyme derivative will generally have the enzymatic activity of the parent enzyme and a collagen derivative at least one structural, chemical or biological property of the parent collagen.

*Biofabricated Leather.* Any type of collagen, truncated collagen, unmodified or post-translationally modified, or amino acid sequence-modified collagen that can be fibrillated and crosslinked by the methods described herein can be used to produce a biofabricated material or biofabricated leather. Biofabricated leather may contain a substantially homogenous collagen, such as only Type I or Type III collagen, or may contain mixtures of 2, 3, 4 or more different kinds of collagens. In some embodiments, a recombinant collagen, for example, a component of a biofabricated leather, will have none of its lysine, proline, or lysine and proline residues hydroxylated. In others at least 1, 2, 3, 4, 5, 10, 15, 20, 30, 40, 50, 60, 70, 80, 90, 95% or 100% (or any intermediate value of subrange) of the lysine, proline, or lysine and proline residues in a recombinant collagen will be hydroxylated.

*Yeast strains.* The present disclosure utilizes yeast to produce collagen. Suitable yeasts include, but are not limited to, those of the genus *Pichia, Candida, Komatagaella, Hansenula, Saccharomyces, Cryptococcus, Arxula, Ogataea* and combinations thereof. The yeast may be modified or hybridized. Hybridized yeasts are produced by mixed breeding of different strains of the same species, different species of the same genus, or strains of different genera. Some yeast strains that may be used according to the disclosure include *Pichia pastoris, Pichia membranifaciens, Pichia deserticola, Pichia cephalocereana, Pichia eremophila, Pichia myanmarensis, Pichia anomala, Pichia nakasei, Pichia siamensis, Pichia heedii, Pichia barkeri, Pichia norvegensis, Pichia thermomethanolica, Pichia stipites, Pichia subpelliculosa, Pichia exigua, Pichia occidentalis,* and *Pichia cactophila.*

In one embodiment, the invention is directed to *Pichia pastoris* strains according to claim 4 that have been engineered to express codon-modified polynucleotides that encode bovine collagen and optionally hydroxylase(s). Useful *Pichia pastoris* host strains include, but are not limited to, BG10 (wild type)(Strain PPS-9010); BG 11, aox1Δ (MutS)(Strain PPS-9011) which is a slow methanol utilization derivative of PPS-9010; and BG16, pep4Δ, prb4Δ (Strain PPS-9016) which is protease deficient. These strains are publically available and may be obtained from ATUM at https://www._atum. bio/products/cell-strains.

*Polypeptide secretion sequences for yeast.* In some embodiments, a polypeptide encoded by a yeast host cell will be fused to a polypeptide sequence that facilitates its secretion from the yeast, for example, a vector may encode a chimeric gene comprising a coding sequence for collagen fused to a sequence encoding a secretion peptide. Secretion sequences which may be used for this purpose include *Saccharomyces* alpha mating factor Prepro sequence, *Saccharomyces* alpha mating factor Pre sequence, PHO1 secretion signal, α-amylase signal sequence from *Aspergillus niger,* Glucoamylase signal sequence from *Aspergillus awamori,* Serum albumin signal sequence from *Homo sapiens,* Inulinase signal sequence from *Kluyveromcyes maxianus,* Invertase signal sequence from *Saccharomyces cerevisiae,* Killer protein signal sequence from *Saccharomyces cerevisiae* and Lysozyme signal sequence from *Gallus gallus.* Other secretion sequences known in the art may also be used.

*Yeast promoters and terminators.* In some embodiments one or more of the following yeast promoters may be incorporated into a vector to promoter transcription of mRNA encoding collagen or a hydroxylase. Promoters are known in the art and include pAOX1, pDas1, pDas2, pPMP20, pCAT, pDF, pGAP, pFDH1, pFLD1, pTAL1, pFBA2, pAOX2, pRKI1, pRPE2, pPEXS, pDAK1, pFGH1, pADH2, pTPI1, pFBP1, pTAL1, pPFK1, pGPM1, and pGCW14.

In some embodiments a yeast terminator sequence is incorporated into a vector to terminate transcription of mRNA encoding collagen or a hydroxylase. Terminators include but are not limited to AOX1 TT, Das1 TT, Das2 TT, AOD TT, PMP TT, Cat1 TT, TPI TT, FDH1 TT, TEF1 TT, FLD1 TT, GCW14 TT, FBA2 TT, ADH2 TT, FBP1 TT, and GAP TT.

*Peptidases other than pepsin.* Pepsin may be used to process collagen into tropollagen by removing N-terminal and C-terminal sequences. Other proteases, including but not limited to collagenase, trypsin, chymotrypsin, papain, ficain, and bromelain, may also be used for this purpose. As used herein, "stable collagen" means that after being exposed to a particular concentration of pepsin or another protease that at least 20, 30,40, 50, 60, 75, 80, 85, 90, 95 or 100% (or any intermediate value or subrange) of the initial concentration of collagen is still present. Preferably, at least 75% of a stable collagen will remain after treatment with pepsin or another protease as compared to an unstable collagen treated under the same conditions for the same amount of time. Prior to post-translational modification, collagen is non-hydroxylated and degrades in the presence of a high pepsin concentration (e.g., a pepsin:protein ratio of 1:200 or more).

Once post-translationally modified a collagen may be contacted with pepsin or another protease to cleave the N-terminal and the C-terminal propeptides of collagen, thus enabling collagen fibrillation. Hydroxylated collagen has better thermostability compared to non-hydroxylated collagen and is resistant to high concentration pepsin digestion, for example at a pepsin:total protein ratio of 1:25, 1:20, 1:15, 1:10, 1:5, to 1:1 (or any intermediate value). Therefore, to avoid premature proteolysis of recombinant collagen it is useful to provide hydroxylated collagen.

*Alternative expression systems.* Collagen can be expressed in other kinds of yeast cells besides *Pichia pastoris,* for example, in may be expressed in another yeast, methylotrophic yeast or other organism. *Saccharomyces cerevisiae* can be used with any of a large number of expression vectors. Commonly employed expression vectors are shuttle vectors containing the 2P origin of replication for propagation in yeast and the Col E1 origin for *E. coli,* for efficient transcription of the foreign gene. A typical example of such vectors based on 2P plasmids is pWYG4, which has the 2P ORI-STB elements, the GAL1-10 promoter, and the 2P D gene terminator. In this vector, a Ncol cloning site is used to insert the gene for the polypeptide to be expressed and to provide an ATG start codon.

Another expression vector is pWYG7L, which has intact 2αORI, STB, REP1 and REP2, and the GAL1-10 promoter, and uses the FLP terminator. In this vector, the encoding polynucleotide is inserted in the polylinker with its 5' ends at a BamHl or Ncol site. The vector containing the inserted polynucleotide is transformed into *S. cerevisiae* either after removal of the cell wall to produce spheroplasts that take up DNA on treatment with calcium and polyethylene glycol or by treatment of intact cells with lithium ions.

Alternatively, DNA can be introduced by electroporation. Transformants can be selected, for example, using host yeast cells that are auxotrophic for leucine, tryptophan, uracil, or histidine together with selectable marker genes such as LEU2, TRP1, URA3, HIS3, or LEU2-D.

There are a number of methanol responsive genes in methylotrophic yeasts such as *Pichia pastoris,* the expression of each being controlled by methanol responsive regulatory regions, also referred to as promoters. Any of such methanol responsive promoters are suitable for use in the practice of the present invention. Examples of specific regulatory regions include the AOX1 promoter, the AOX2 promoter, the dihydroxyacetone synthase (DAS), the P40 promoter, and the promoter for the catalase gene from *P. pastoris, etc.*

The methylotrophic yeast *Hansenula polymorpha* may also be employed. Growth on methanol results in the induction of key enzymes of the methanol metabolism, such as MOX (methanol oxidase), DAS (dihydroxyacetone synthase), and FMHD (formate dehydrogenase). These enzymes can constitute up to 30-40% of the total cell protein. The genes encoding MOX, DAS, and FMDH production are controlled by strong promoters induced by growth on methanol and repressed by growth on glucose. Any or all three of these promoters may be used to obtain high-level expression of heterologous genes in *H. polymorpha.* Therefore, in one aspect, a polynucleotide encoding animal collagen or fragments or variants thereof is cloned into an expression vector under the control of an inducible *H. polymorpha* promoter. If secretion of the product is desired, a polynucleotide encoding a signal sequence for secretion in yeast is fused in frame with the polynucleotide. In a further embodiment, the expression vector preferably contains an auxotrophic marker gene, such as URA3 or LEU2, which may be used to complement the deficiency of an auxotrophic host.

The expression vector is then used to transform *H. polymorpha* host cells using techniques known to those of skill in the art. A useful feature of *H. polymorpha* transformation is the spontaneous integration of up to 100 copies of the expression vector into the genome. In most cases, the integrated polynucleotide forms multimers exhibiting a head-to-tail arrangement. The integrated foreign polynucleotide has been shown to be mitotically stable in several recombinant strains, even under non-selective conditions. This phenomena of high copy integration further ads to the high productivity potential of the system.

Foreign DNA is inserted into the yeast genome or maintained episomally to produce collagen. The DNA sequence for the collagen is introduced into the yeast via a vector. Foreign DNAs are any non-yeast host DNA and include for example, but not limited to those from mammals, *Caenorhabditis elegans* and bacteria. Suitable mammalian DNA for collagen production in yeast include, but is not limited to, bovine, equine, porcine, kangaroo,, elephant, rhinoceros, hippopotamus, whale, dolphin, giraffe, zebra, llama, alpaca, goat, and sheep (lamb). Other DNAs for collagen production include those from reptiles (such as alligator, crocodile, turtle, iguana, lizard, snake), avian *(e.g*., ostrich, emu, moa), dinosaurs, amphibians, and fish *(e.g*., tilapia, bass, salmon, trout, shark, eel collagen) and combinations thereof.

DNA is inserted on a vector, suitable vectors include, but are not limited to, pHTX1-BiDi-P4HA-Pre-P4HB hygro, pHTX1- BiDi-P4HA-PHO1-P4HB hygro, pGCW14-pGAP1- BiDi-P4HA-Prepro-P4HB G418, pGCW14-pGAP1- BiDi-P4HA-PHO1-P4HB Hygro, pDF-Col3A1 modified Zeocin, pCAT-Col3A1 modified Zeocin, pDF-Col3A1 modified Zeocin with AOX1 landing pad, pHTX1- BiDi-P4HA-Pre-Pro-P4HB hygro. The vectors typically included at least one restriction site for linearization of DNA.

A select promoter may improve the production of a recombinant protein and may be included in a vector comprising sequences encoding collagen or hydroxylates. Suitable promoters for use in the present invention include, but are not limited to, AOX1 methanol induced promoter, pDF de-repressed promoter, pCAT de-repressed promoter, Das1-Das2 methanol induced bi-directional promoter, pHTX1 constitutive Bi-directional promoter, pGCW14-pGAP1 constitutive Bi-directional promoter and combinations thereof. Suitable methanol induced promoters include but are not limited to AOX2, Das 1, Das 2, pDF, pCAT, pPMP20, pFDH1, pFLD1, pTAL2, pFBA2, pPEXS, pDAK1, pFGH1, pRKI1, pREP2 and combinations thereof.

In the vectors comprising the chimeric bovine collagen DNA sequence of claim 1 according to the invention, including the all-in-one vector, a terminator may be placed at the end of each open reading frame utilized in the vectors incorporated into the yeast. The DNA sequence for the terminator is inserted into the vector. For replicating vectors, an origin of replication is necessary to initiate replication. The DNA sequence for the origin of replication is inserted into the vector. One or more DNA sequences containing homology to the yeast genome may be incorporated into the vector to facilitate recombination and incorporation into the yeast genome or to stabilize the vector once transformed into the yeast cell.

A vector comprising the chimeric bovine collagen DNA sequence of claim 1 according to the invention will also generally include at least one selective marker that is used to select yeast cells that have been successfully transformed. The markers sometimes are related to antibiotic resistance and markers may also be related to the ability to grow with or without certain amino acids (auxotrophic markers). Suitable auxotrophic markers included, but are not limited to ADE, HIS, URA, LEU, LYS, TRP and combinations thereof. To provide for selection of yeast cells containing a recombinant vector, at least one DNA sequence for a selection marker is incorporated into the vector.

In some embodiments of the disclosure, amino acid residues, such as lysine and proline, in a recombinant yeast-expressed collagen or collagen-like protein may lack hydroxylation or may have a lesser or greater degree of hydroxylation than a corresponding natural or unmodified collagen or collagen-like protein. In other embodiments, amino acid residues in a collagen or collagen-like protein may lack glycosylation or may have a lesser or greater degree of glycosylation than a corresponding natural or unmodified collagen or collagen-like protein.

Hydroxylated collagen has a higher melting temperature (>37°C) than non-hydroxylated or under hydroxylated collagen (<32°C) and also fibrillates better than non-hydroxylated or under hydroxylated collagen and forms stronger more durable structures for use as materials. The melting temperature of a collagen preparation may be used to estimate its degree of hydroxylation and can range, for example, from 30 to 40°C, as well as all intermediate values such as 30, 31, 32, 33, 34, 35, 36, 37, 38, 39, and 40°C. Under hydroxylated collagen may only form a jello- or gelatin-like material not suitable for durable items such as shoes or bags but which can be formulated into softer or more absorbent products.

The collagen in a collagen composition may be homogenous and contain a single type of collagen molecule, such as 100% bovine Type I collagen or 100% Type III bovine collagen, or may contain a mixture of different kinds of collagen molecules or collagen-like molecules, such as a mixture of bovine Type I and Type III molecules. Such mixtures may include >0%, 10, 20, 30, 40, 50, 60, 70, 80, 90, 95, 99 or <100% (or any intermediate value or subrange) of the individual collagen or collagen-like protein components. This range includes all intermediate values. For example, a collagen composition may contain 30% Type I collagen and 70% Type III collagen, or may contain 33.3% of Type I collagen, 33.3% of Type II collagen, and 33.3% of Type III collagen, where the percentage of collagen is based on the total mass of collagen in the composition or on the molecular percentages of collagen molecules.

The engineered yeast cells described above can be utilized to produce collagen. In order to do so, the cells are placed in media within a fermentation chamber and fed dissolved oxygen and a source of carbon, under controlled pH conditions for a period of time ranging from twelve hours to 1 week. Suitable media include but are not limited to buffered glycerol complex media (BMGY), buffered methanol complex media (BMMY), and yeast extract peptone dextrose (YPD). Due to the fact that collagen is produced in the yeast cell, in order to isolate the collagen, one must either use a secretory strain of yeast or lyse the yeast cells to release the collagen. The collagen may then be purified through conventional techniques such as centrifugation, precipitation, filtration, chromatography, and the like.

In another embodiment, the invention provides chimeric DNA sequences according to claim 1 in yeast hosts that are useful for producing hydroxylated and non-hydroxylated collagen. Chimeric DNA sequences are produced by combining unmodified and modified DNA sequences. The unmodified DNA sequence may be cut at various base pair locations. The modified DNA sequence may also be cut at corresponding base pair locations. The unmodified and modified cuts may be combined front to back and back to front. The chimeric DNA sequences may be combined with promoters, vectors, terminators and selection markers from above and inserted into a host to generate yeast that can produce hydroxylated and non-hydroxylated collagen.

The percent of optimized and unoptimized DNA may be calculated based on the total length of the sequence. The chimera strain is a combination of optimized DNA encoding the amino acid sequence at the N-terminus and unoptimized DNA encoding the amino acid sequence at the C-terminus of a bovine collagen molecule, as outlined in claimed in claim 1. The percent of optimized DNA ranges from 60 to 90%. The percent of unoptimized DNA ranges from 10 to 40%. For example, a DNA sequence with 1486 base pairs cut at 1331 will provide 0-1331 optimized DNA and 1332-1486 unoptimized DNA and the chimera will be 90% optimized. An optimized polynucleotide sequence encodes a segment of collagen at the the N-terminus.

According to the disclosure, the chimeric strain may be made up of two, three or four or more sections of optimized and unoptimized DNA fused together. For example, a DNA sequence with 1,500 base pairs may have an optimized DNA section from 0 to 500, an unoptimized DNA from 501 to 1,000 and an optimized DNA section from 1001 to 1500.

The collagen disclosed herein makes it possible to produce a biofabricated leather. Methods for converting collagen to biofabricated leather are taught in co-pending patent applications U.S. application numbers 15/433566, 15/433650, 15/433632, 15/433693, 15/433777, 15/433675, 15/433676 and 15/433877.

### EMBODIMENTS OF THE INVENTION AND THE DISCLOSURE

Non-limiting embodiments of the invention and the disclosure include but are not limited to:
A polynucleotide of the disclosure, which encodes bovine collagen, such as Type I or Type III collagen, or a collagen variant or derivative and at least one enzyme that hydroxylates proline, lysine, or lysine and proline residues in the encoded collagen. In some embodiments the polynucleotide will codon- modify all or part of the native collagen or hydroxylase polynucleotide sequences or incorporate expression control elements such as yeast promoter sequences to facilitate expression of the collagen or hydroxylase in a host yeast cell. The modified polynucleotide when expressed in yeast may increase collagen expression by comparison to an unmodified polypeptide expressed under identical conditions that encodes the same collagen sequence by 10, 20, 25, 30, 35, 40, 45, 50, 60, 70, 80, 90, 100 or >100 wt%.

In some embodiments of the disclosure, 2-, 3-, 4-, 5-, 6-, 7-, 8-, 9-, 10-, 11-, 12-, 13-, 14-, 15- or greater-fold expression of collagen or hydroxylase proteins may be attained. In some embodiments a Type III collagen or collagen variant will be expressed, where the variant has an amino acid sequence that is at least 70, 75, 80, 85, 90, 95, 96, 97, 98, 99 or 100% identical to that of SEQ ID NO: 2. In other embodiments the bovine collagen is a Type I bovine collagen or collagen variant which encodes both α1(I) chains and an α2(I) chain or that encodes one or more collagen chains that is at least 70, 75, 80, 85, 90, 95, 96, 97, 98, 99 or 100% identical to the native Type I collagen chains.

The polynucleotide encoding bovine collagen of the disclosure described above may include a polynucleotide sequence or segment that encodes the P4HA and P4HB subunits of prolyl 4-hydroxylase or a polynucleotide sequence that encodes an enzyme that is at least 70, 75, 80, 85, 90, 95, 96, 97, 98, 99 or 100% identical thereto. In other embodiments the polynucleotide can contain a polynucleotide sequence or segment that encodes prolyl-3-hydroxylase, lysyl hydroxylase, and/or lysyl oxidase or a polynucleotide sequence that encodes an enzyme that is at least 70, 75, 80, 85, 90, 95, 96, 97, 98, 99 or 100% identical thereto. For example, a polynucleotide of the disclosure can encode a polypeptide that is at least 75-99% identical to the Type III bovine collagen amino acid sequence of SEQ ID NO: 2 and a segment that encodes a hydroxylase comprising P4HA and P4HB subunits that are at least 75-99% identical to SEQ ID NOS: 54 and 52, respectively.

A polynucleotide sequence of the invention may further encode a polypeptide secretion sequence operative in yeast which is generally placed adjacent to a polynucleotide sequence being the chimeric bovine collagen DNA sequence according to claim 1.

A polynucleotide sequence of the invention may further contain a promoter or other sequence that facilitates or controls expression of collagen or enzymes, such as hydroxylases, for example, it may contain at least one of an AOX1 methanol induced promoter, DN pDF de-repressed promoter, pCAT de-repressed promoter, Das1-Das2 methanol induced bi-directional promoter, pHTX1 constitutive Bi-directional promoter, pGCW14-pGAP1 constitutive Bi-directional promoter, or combinations thereof.

A polynucleotide of the invention may also contain other elements such as an alpha factor pre- or alpha factor pre-pro sequence such as those respectively encoded by SEQ ID NOS: 23 and 24. In some embodiments, such a sequence may be operatively linked to a polynucleotide sequence that expresses an enzyme, such as a hydroxylase or other enzymes described herein such as P4HA (SEQ ID NO: 54) or P4HB (SEQ ID NO: 52), or to a variant enzyme that is at least 75, 80, 90, or 95-100% identical thereto.

Vectors containing the polynucleotide sequences of claim 1 and optionally the additional embodiments disclosed above represent additional embodiments of the invention which are referred to in claim 4. These correspond to a vector that contains the chimeric bovine collagen DNA sequence of claim 1. In some embodiments the sequence encoding the bovine collagen of claim 1 and the sequence encoding a hydroxylase or other enzyme will be on the same vector; in others they may be on different vectors.

The invention also contemplates yeast host cells that contain the vectors described herein, namely the strain according to claim 4. In some embodiments, these vectors may be produced in non-yeast cells, such as in bacterial host cells and later transformed into yeast host cells, such as *Pichia pastoris* host cells, that express collagen or hydroxylated collagen.

Another aspect of the disclosure is directed to a method for producing recombinant collagen which has less than 1, 2, 3, 4, 5, 6, 7, 8, 9, or 10% of its proline residues hydroxylated. This method involves culturing a *Pichia pastoris* or another suitable yeast host cell (or eukaryotic host cell) for a time and under conditions suitable for producing collagen, and recovering the collagen; wherein said vector is configured to express an amount or form of prolyl-4-hydroxylase that hydroxylates no more than 1, 2, 3, 4, 5, 6, 7, 8, 9, 10% of the proline residues. Another embodiment of the disclosure is a method for producing recombinant Type III collagen which has less than 1, 2, 3, 4, 5, 6, 7, 8, 9, 10% of its proline residues hydroxylated involving culturing a *Pichia pastoris* or other suitable yeast host cell for a time and under conditions suitable for producing Type III collagen, and recovering the collagen; wherein said vector is configured to express an amount of prolyl-4-hydroxylase that hydroxylates no more than 1, 2, 3, 4, 5, 6, 7, 8, 9, 10% of the proline residues. An all-in-one vector that encodes both collagen and a hydroxylase may be configured so that little or no functional hydroxylase is expressed, *e.g.*, by use of an inducible or temperature sensitive promoter for the hydroxylase.

A further embodiment of the disclosure is a method for producing recombinant collagen which has >10, 15, 20, 25, 30, 40, 50, 60, 70, 80, 90, 95 or >95% of its proline residues hydroxylated by culturing *Pichia pastoris* or another suitable yeast host cell containing a vector as described herein for a time and under conditions suitable for producing collagen, and recovering the collagen; wherein the vector is configured to express an amount or form of prolyl-4-hydroxylase that hydroxylates >10, 15, 20,25, 30, 40, 50, 60, 70, 80, 90 or >90% or more of the proline residues in the collagen. The culture time and conditions and the amount or activity of the hydroxylase may be used to control the amount of hydroxylation. Another embodiment of the disclosure is a method for producing recombinant Type III collagen which has 50, 60, 70, 80, 90, 95, or >95% of its proline residues hydroxylated comprising culturing a *Pichia pastoris* host cell containing a vector according to the discosure for a time and under conditions suitable for producing Type III collagen, and recovering the Type III collagen; wherein the vector is configured to express an amount or form of prolyl-4-hydroxylase that hydroxylates 50, 60, 70, 80, 90 or >90% or more of the proline residues. The culture time and conditions and the amount or activity of the hydroxylase may be used to control the amount of hydroxylation.

Another embodiment of the disclosure is directed to a method for producing recombinant collagen which has 50, 60, 70, 80, 90, 95 or >95% or more of its proline residues hydroxylated comprising culturing the *Pichia pastoris* or other suitable yeast host cell containing a vector of the disclosure for a time and under conditions suitable for producing collagen, and recovering the collagen; wherein the vector is configured to express an amount of prolyl-4-hydroxylase that hydroxylates 50, 60, 70, 80, 90, 95, or >95% or more of the proline residues. The culture time and conditions and the amount or activity of the hydroxylase may be used to control the amount of hydroxylation.

Another embodiment of the disclosure is directed to a method for producing recombinant Type III collagen which has 50, 60, 70, 80, 90, 95, or >95% of its proline residues hydroxylated comprising culturing a *Pichia pastoris* or other yeast host cell containing a vector of the disclosure for a time and under conditions suitable for producing collagen, and recovering the collagen; wherein said vector is configured to express an amount of prolyl-4-hydroxylase that hydroxylates 50, 60, 70, 80, 90, 95, or >95% of the proline residues. The culture time and conditions and the amount or activity of the hydroxylase may be used to control the amount of hydroxylation.

Another embodiment of the disclosure is directed to a method for producing recombinant collagen which has 75, 80, 90, 95, or >95% of its proline residues hydroxylated including culturing the *Pichia pastoris* or other yeast host cell containing a vector of the disclosure for a time and under conditions suitable for producing collagen, and recovering the collagen; wherein said vector is configured to express an amount of prolyl-4-hydroxylase that hydroxylates 75, 80, 90, 95, or >95% of the proline residues.

A further embodiment of the disclosure is a method for producing recombinant Type III collagen which has 75, 80, 90, 95, or >95% of its proline residues hydroxylated comprising culturing the *Pichia pastoris* or other yeast host cell containing a vector of the disclosure for a time and under conditions suitable for producing collagen, and recovering the collagen; wherein said vector is configured to express an amount of prolyl-4-hydroxylase that hydroxylates 75, 80, 90, 95, or >95% or more of the proline residues.

Another embodiment of the disclosure is a recombinant collagen made by any one of the methods described herein. Such a recombinant collagen may have none of its proline or lysine residues hydroxylated or may have >0, 10, 20, 30, 40, 50, 60, 70, 80, 90, 95, or 100% of the proline, lysine or proline and lysine residues hydroxylated.

A further embodiment of the disclosure is a biofabricated leather or other material comprising the recombinant collagen as described herein or which is made by a method described herein.

In another embodiment, the invention provides chimeric DNA sequences as claimed in claim 1 in yeast host cells that are useful for producing hydroxylated and unhydroxylated collagen. Chimeric DNA sequences are produced by combining unmodified and modified DNA sequences as set out in claim 1. The unmodified DNA sequence may be cut at various base pair locations. The modified DNA sequence may also be cut at corresponding base pair locations. The unmodified and modified cuts may be combined front to back and back to front. The chimeric DNA sequences may be combined with promoters, vectors, terminators and selection markers from above and inserted into a host to generate yeast that can produce hydroxylated and non-hydroxylated collagen.

Other embodiments of the invention and the disclosure, include but are not limited to:
The disclosure provides a strain of yeast genetically engineered to produce non-hydroxylated collagen including (i) a strain of yeast; and (ii) a vector comprising a DNA sequence for collagen; a DNA sequence for a collagen promotor; a DNA sequence for a collagen terminator; a DNA sequence for a selection marker, a DNA sequence for a promoter for the selection marker; a DNA sequence for a terminator for the selection marker; a DNA sequence for a replication origin selected from one for bacteria and one for yeast; and a DNA sequence containing homology to the yeast genome, wherein the vector has been inserted into the strain of yeast. In this embodiment, the strain of yeast may be selected from the group consisting of those from the genus *Pichia, Candida, Komatagaella, Hansenula, Saccharomyces, Cryptococcus, Arxula, Ogataea* and combinations thereof. In the above embodiment, the vector may contain a DNA sequence for collagen selected from the group consisting of bovine, porcine, kangaroo, alligator, crocodile, elephant, giraffe, zebra, llama, alpaca, lamb, dinosaur collagen, and combinations thereof. In this embodiment, the DNA sequence for collagen may be selected from native collagen DNA, engineered collagen DNA, and codon modified collagen DNA.

In this embodiment, the DNA sequence for the promotor can be selected from the group consisting of DNA for the AOX1 methanol induced promoter, DNA for the pDF de-repressed promoter, DNA for the pCAT de-repressed promoter, DNA for the Das1-Das2 methanol induced bi-directional promoter, DNA for the pHTX1 constitutive Bi-directional promoter, DNA for the pGCW14-pGAP1 constitutive Bi-directional promoter and combinations thereof. The selection marker in this embodiment may be selected from the group consisting of a DNA for antibiotic resistance and a DNA for auxotrophic marker, for example, the antibiotic resistance may be to an antibiotic selected from the group consisting of hygromycin, zeocin, geneticin and combinations thereof.

The yeast strain as described in the above embodiment may contain a vector that was inserted into the yeast through a method selected from the group consisting of electroporation, chemical transformation, and mating.

Another embodiment of the disclosure is directed to a method for producing non-hydroxylated collagen including (i) providing a strain of yeast as described by the embodiments above; and (ii) growing the strain in a media for a period of time sufficient to produce collagen. In this method the yeast may be selected from the group consisting of those from the genus *Pichia, Candida, Komatagaella, Hansenula, Saccharomyces, Cryptococcus, Arxula, Ogataea* and combinations thereof and/or the medium selected from the group consisting of buffered glycerol complex media (BMGY), buffered methanol complex media (BMMY), and yeast extract peptone dextrose (YPD). The yeast strain may be cultured or cultivated for a period of time ranging from 24, 48, or 72 hours or any intermediate time period. In particular, this period of time is from 24 hours to 72 hours. In this method the yeast strain may express a DNA sequence for collagen selected from the group consisting of bovine, porcine, kangaroo, alligator, crocodile, elephant, giraffe, zebra, llama, alpaca, lamb, dinosaur collagen and combinations thereof. In this method, the DNA sequence for the promoter in the yeast strain may be selected from the group consisting of the DNA for pHTX1 constitutive Bi-directional promoter and the DNA for pGCW14-pGAP1 constitutive Bi-directional promoter; and/or the selection marker may be selected from the group consisting of the DNA for antibiotic resistance DNA and the DNA for the auxotrophic marker.

Another embodiment of the disclosure is a strain of yeast genetically engineered to produce hydroxylated collagen that includes (i) a strain of yeast and (ii) a vector containing a DNA sequence for collagen; a DNA sequence for a collagen promotor; a DNA sequence for a terminator; a DNA sequence for a selection marker; a DNA sequence for a promoter for the selection marker; a DNA sequence for a terminator for the selection marker; a DNA sequence for a replication origin for bacteria and/or yeast; a DNA sequence containing homology to the yeast genome; wherein the vector has been inserted into the strain of yeast; and (iii) a second vector comprising a DNA sequence for P4HA1; a DNA sequence for P4HB; and at least one DNA sequence for a promoter, wherein the vectors have been inserted into the strain of yeast. In this embodiment, the yeast strain may be selected from the group consisting of those from *the genus Pichia, Candida, Komatagaella, Hansenula, Saccharomyces, Cryptococcus, Arxula, Ogataea* and combinations thereof; and/ or the yeast strain may express a DNA sequence for collagen selected from the group consisting of bovine, porcine, kangaroo, alligator, crocodile, elephant, giraffe, zebra, llama, alpaca, lamb, dinosaur collagen and combinations thereof. In the strain of yeast of this embodiment the DNA sequence for collagen is selected from native collagen DNA, engineered collagen DNA and modified collagen DNA; and/or the DNA sequence for the promoter is selected from the group consisting of DNA for the AOX1 methanol induced promoter, DNA for the pDF de-repressed promoter, DNA for the pCAT de-repressed promoter, DNA for the Das1-Das2 methanol induced bi-directional promoter, DNA for the pHTX1 constitutive Bi-directional promoter, DNA for the pGCW14-pGAP1 constitutive Bi-directional promoter and combinations thereof. In the strain of yeast of this embodiment, the DNA sequence for the promoter can be selected from the group consisting of the DNA for pHTX1 constitutive Bi-directional promoter and the DNA for pGCW14-pGAP1 constitutive Bi-directional promoter; and/or the DNA sequence for the selection marker can be selected from the group consisting of the DNA for the antibiotic resistance DNA and the DNA for the auxotrophic marker. Some examples of antibiotic resistance genes or DNA include resistance to and antibiotic selected from the group consisting of hygromycin, zeocin, geneticin and combinations thereof, though other known antibiotic resistance genes may also be used. The vector may be inserted into the strain of yeast of this embodiment through a method selected from the group consisting of electroporation, chemical transformation, and mating.

Another embodiment of the disclosure is a method for producing hydroxylated collagen that includes (i) providing a strain of yeast as described herein i.e. a strain of yeast genetically engineered to produce hydroxylated collagen, and (ii) growing the strain in a media for a period of time sufficient to produce collagen. The strain of yeast can be selected from the group consisting of those from the genus *Pichia, Candida, Komatagaella, Hansenula, Saccharomyces, Cryptococcus, Arxula, Ogataea* and combinations thereof; the collagen DNA expressed by the yeast strain may be selected from the group consisting of DNA encoding bovine, porcine, kangaroo, alligator, crocodile, elephant, giraffe, zebra, llama, alpaca, lamb, dinosaur collagen or combinations thereof; and/or the medium selected from the group consisting of BMGY, BMMY, and YPD. The yeast strain may be cultured or cultivated for a period of time ranging from about 24, 48 or 72 hours. In particular, this period of time is from 24 hours to 72 hours. In the method of this embodiment, the DNA for the promotor is selected from the group consisting of the DNA for pTHX1 constitutive Bi-directional promoter and the DNA for pGCW14-pGAP1 constitutive Bi-directional promoter; and/or the DNA for the selection marker is selected from the group consisting of the DNA for the antibiotic resistance DNA and the DNA for the auxotrophic marker.

Another embodiment of the disclosure is directed to an all-in-one vector that includes (i) a DNA that when expressed produces collagen, a promoter, and a terminator; (ii) at least one DNA for one or more hydroxylation enzymes selected from the group consisting of P4HA1 and P4HB, including promoters and terminators; (iii) at least one DNA for a selection marker; including a promoter and a terminator; (iv) at least one DNA for an origin or origins of replication for yeast and bacteria; (v) one or more DNAs with homology to the yeast genome for integration into the genome; and (iv) one or more restriction sites at a position selected from the group consisting of 5', 3', within the above DNAs, and combinations thereof allowing for modular cloning. In some embodiments, the all-in-one vector will contain one or more DNA sequences that when expressed produce a collagen selected from the group consisting of bovine, porcine, kangaroo, alligator, crocodile, elephant, giraffe, zebra, llama, alpaca, lamb, dinosaur collagen and combinations thereof.

The all-in-one vector may include a promoter selected from the group consisting of the DNA for pTHX1 constitutive Bi-directional promoter and the DNA for pGCW14-pGAP1 constitutive Bi-directional promoter and/or may include one or more DNA sequences for selection markers, such as antibiotic resistance and/or auxotrophic markers. Antibiotic resistance markers include resistance to an antibiotic selected from the group consisting of hygromycin, zeocin, geneticin and combinations thereof.

Another embodiment of the invention is directed to a chimeric bovine collagen DNA sequence, that contains from 60 to 90 percent of optimized DNA based on the total length of the chimeric collagen DNA as claimed in claim 1. In this chimeric collagen DNA sequence the optimized DNA originates at the N-terminus, i.e. the optimized DNA encodes the amino acid sequence at the N-terminus of a bovine collagen molecule, as claimed in claim 1. In an embodiment of the disclosure, the chimeric collagen DNA sequence may encode Type I collagen and have been codon-optimized for expression in *Pichia pastoris*.According to the invention, the chimeric collagen DNA sequence encodes bovine collagen, wherein the DNA sequence is as claimed in claim 1.. The chimeric collagen DNA sequence of this embodiment may further comprise a polynucleotide sequence encoding P4HA1 and/or PAHB.

Another embodiment of the invention is directed to strain of collagen-producing yeast that includes a vector comprising a DNA sequence for a chimeric collagen as claimed in claim 1 and as further described herein, wherein the strain is claimed in claim 4; a DNA sequence for a collagen promotor; a DNA sequence for a terminator; a DNA sequence for a selection marker; a DNA sequence for a promoter for the selection marker; a DNA sequence for a terminator for the selection marker; a DNA sequence for a replication origin for bacteria and/or yeast; and a DNA sequence containing homology to the yeast genome. In this embodiment, the strain of yeast may contain a DNA for the promoter selected from the group consisting of the DNA for pTHX1 constitutive Bi-directional promoter and the DNA for pGCW14-pGAP1 constitutive Bi-directional promoter. The strain of yeast may contain a selection marker selected from the group consisting of the DNA encoding at least one antibiotic resistance and DNA encoding at least one auxotrophic marker.

Another embodiment of the invention is directed to a method for producing hydroxylated collagen as claimed in claim 6 that includes (i) providing a strain of yeast as described herein i.e. a strain of collagen-producing yeast that includes a vector comprising a DNA sequence for a chimeric collagen as claimed in claim 1 and as further described herein; and (ii) growing the strain in a medium for a period of time sufficient to produce collagen, wherein the period is 24 hours to 72 hours. In this embodiment, the strain of yeast can selected from the group consisting of those from the genus *Pichia, Candida, Komatagaella, Hansenula, Saccharomyces, Cryptococcus, Arxula, Ogataea* and combinations thereof; the medium may be selected from the group consisting of buffered glycerol complex media, buffered methanol complex media, and yeast extract peptone dextrose; and culture or cultivation time is 24, 48 or 72 hours and generally ranges from 24 hours to 72 hours. In some embodiments of this method, the strain of yeast includes a promoter selected from the group consisting of the DNA for pTHX1 constitutive Bi-directional promoter and the DNA for pGCW14-pGAP1 constitutive Bi-directional promoter. In other embodiments of this method, the strain of yeast comprises at least one selection marker selected from the group consisting of DNA encoding an antibiotic resistance and DNA encoding an auxotrophic marker.

### EXAMPLES

The following non-limiting Examples are illustrative of the present invention. The scope of the invention is not limited to the details described in these Examples.

### EXAMPLE 1

*Pichia pastoris* strain BG10 (wild type) was obtained from ATUM (formerly DNA 2.0). A MMV 63 (SEQ ID NO: 11) ("Sequence 9") DNA sequence including a collagen sequence and vectors, were inserted into wild type *Pichia pastoris* which generated strain PP28. MMV63 was digested by Pme I and transformed into PP1 (Wild Type *Pichia pastoris* strain) to generate PP28. The vector MMV63 is shown in FIG. 1.

DNA encoding native Type III bovine collagen was sequenced (SEQ ID NO: 1) and the sequence was amplified by polymerase chain reaction "PCR" protocol to create a linear DNA sequence.

The DNA was transformed into wild-type *Pichia* yeast cells (PP1) from DNA 2.0 using a *Pichia* Electroporation Protocol (Bio-Rad Gene Pulser Xcell^{™} Total System #1652660). Yeast cells were transformed with P4HA/B co-expression plasmid and transformants (e.g., Clone #4) selected on a Hygro plate (200 ug/ml).

A single colony of Clone #4 was inoculated in 100 ml YPD medium and grown at 30 degrees overnight with shaking at 215 rpm. The next day when the culture reached an OD600 ~3.5 (~3-5 × 10⁷ cells/OD600) it was diluted with fresh YPD to OD600 ~1.7 and grown for another hour at 30°C with shaking at 215 rpm.

The cells were then spun down the cells at 3,500g for 5 min; washed once with water and resuspended in 10 ml 10mM Tris-HCl (pH 7.5), 100 mM LiAc, 10 mM DTT (added fresh), and 0.6 M Sorbitol.

For each transformation, an aliquot of 8 × 10⁸ cells was placed into 8 ml 10mM Tris-HCl (pH 7.5), 100 mM LiAc, 10 mM DTT, 0.6 M Sorbitol and incubated at room temperature for 30 min.

The cells were spun down at 5000g for 5 mins and washed with ice cold 1.5 ml 1M Sorbitol 3 times and resuspended in 80 µl ice cold 1M Sorbitol .

Various amounts (about 5 µg) of linearized DNA were added to the cells and mixed by pipetting.

The cell and DNA mixture (80-100µl) were added into 0.2 cm cuvette and pulsed according to a protocol for *Pichia* at 1500 v, 25 µF, and 200 Ω.

They were then immediately transferred a 1 ml mixture of YPD and 1M Sorbitol (1:1) and incubated at 30° C for > 2 hrs.

The cells were plated at different densities.

Single colonies were inoculated into 2 ml BMGY media in a 24 deep-well plate and grown out for at least 48 hours at 30° C with shaking at 900 rpm. The resulting cells were tested for collagen using cell lysis, SDS-page and pepsin assay following the procedure below.

Yeast cells were lysed in 1x lysis buffer using a Qiagen TissueLyser at a speed of 30 Hz continuously for 1 mins. Lysis buffer was made from 2.5 ml 1 M HEPES (final concentration 50 mM); 438.3 mg NaCl; final concentration 150 mM; 5 ml Glycerol; final concentration 10%; 0.5 ml Triton X-100; final concentration 1%; and 42 ml Millipure water.

The lysed cells were centrifuged at 2,500 rpm for 15 mins on a tabletop centrifuge. The supernatant was retained and pellet discarded.

*SDS-PAGE.* SDS-PAGE in the presence of 2-mercaptoethanol was performed on the supernatant, molecular weight markers, negative control and a positive control. After electrophoresis the gel was removed and stained with Commassie Blue and then destained in water.

*Pepsin assay.* A pepsin assay was performed with the following procedure:
A BCA assay to obtain the total protein of each sample according to the Thermo Scientific protocol was performed before pepsin treatment. The amount of total protein was normalized to the lowest concentration at or above 0.5 mg/ml for all samples.

A 100 µL sample of lysate was placed in a microcentrifuge tube. A master mix was made containing the following: 37% HCl (0.6µL of acid per 100 µL), and

Pepsin stock at 1mg/mL in deionized water. The amount of pepsin added was at a 1:25 ratio pepsin:total protein (weight:weight).

After addition of pepsin, the samples were mixed three times with a pipette and incubated for an hour at room temperature for the pepsin reaction to take place. After an hour, a 1:1 volume of LDS loading buffer containing β-mercaptoethanol was added to each sample and allowed to incubate for 7 minutes at 70°C. After incubation, the samples were spun at 14,000 rpm for 1 minute to remove turbidity.

Then, 18µL from the top of the samples were added onto 3-8% TAE using TAE buffer and run on a gel for 1hr 10 minutes at 150V. Table 1 below reports the results.

### EXAMPLE 2

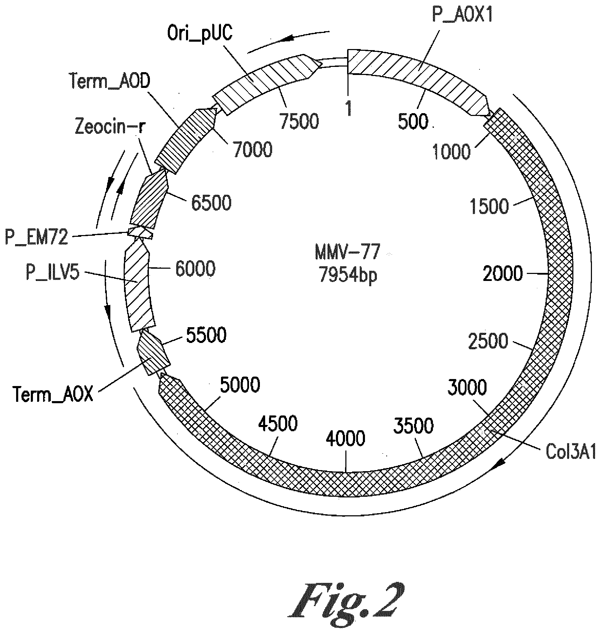

Example 1 was repeated following the same procedures and protocols with the following changes: A DNA MMV77 (SEQ ID NO: 12) ("Sequence 10") sequence including a bovine collagen sequence modified to increase expression in *Pichia* (SEQ ID NO: 3) ("Sequence 2") was inserted into the yeast. A pAOX1 promoter (SEQ ID NO: 5) ("Sequence 3") was used to drive the expression of collagen sequence. A YPD plate containing Zeocin at 500 ug/ml was used to select successful transformants. The resulting strain was PP8. The vector MMV77 is shown in FIG. 2. Restriction digestion was done using Pme I. The strains were grown out in BMMY media and tested for collagen. The results are shown in Table 1 below.

### EXAMPLE 3

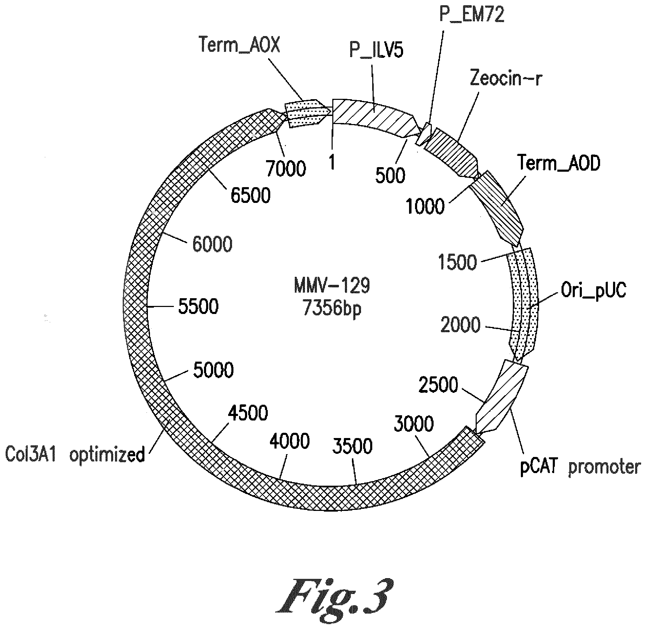

Example 1 was repeated following the same procedures and protocols with the following changes: A DNA MMV-129 (SEQ ID NO: 13)("Sequence 11") sequence including a bovine collagen sequence modified to increase *Pichia* expression was inserted into the yeast. A pCAT promoter (SEQ ID NO: 9) ("Sequence 7") was used to drive the expression of collagen sequence. A YPD plate containing Zeocin at 500 µg/ml was used to select successful transformants. The resulting strain was PP123. MMV129 was digested by Swal and transformed into PP1 to generate PP123. The vector MMV129 is shown in FIG. 3. The strains were grown out in BMGY media and tested for collagen. The results are shown in Table 1 below.

### EXAMPLE 4

Example 1 was repeated following the same procedures and protocols with the following changes:
A DNA MMV-130 (SEQ ID NO: 14) ("Sequence 12") sequence including a bovine Col3A1 (type III) collagen sequence (SEQ ID NO: 3) ("Sequence 2") modified to increase expression in *Pichia* was inserted into the yeast. A pDF promoter shown in SEQ ID NO: 8 ("Sequence 6") was used to drive the expression of collagen sequence. An AOX1 landing pad (SEQ ID NO: 10) ("Sequence 8"), which is cut by Pmel, was used to facilitate site specific integration of the vector into the *Pichia* genome. A YPD plate containing Zeocin at 500 µg/ml was used to select successful transformants. The resulting strain was designated PP153. MMV130 was digested by Pmel and transformed into PP1 to generate PP153. The modified Bovine col3A1 sequence is given by SEQ ID NO: 3 ("Sequence 2").

A PureLink PCR purification kit was used instead of phenol extraction to recover linearized DNA. The strains were grown out in BMGY media and tested for collagen. The results are shown in Table 1 below.

### EXAMPLE 5

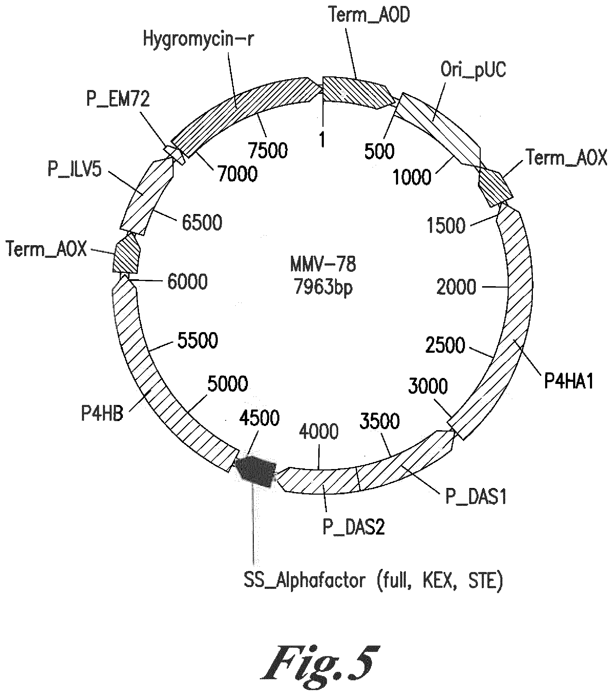

Example 2 was repeated following the same procedures and protocols with the following changes:
One DNA vector, MMV-78 (SEQ ID NO: 15) ("Sequence 13"), containing optimized bovine P4HA (SEQ ID NO: 6) ("Sequence 4") and bovine P4HB (SEQ ID NO: 7)("Sequence 5") sequences were inserted into the yeast. MMV78 was digested by Pmel and transformed into PP1 to generate PP8. Both P4HA and P4HB contained their endogenous signal peptides and are driven by the Das1-Das2 bi-directional promoter (SEQ ID NO: 27) ("Sequence 24"). The DNA was digested by Kpn I and transformed into PP8 to generate PP3. The vector MMV78 is shown in FIG. 5. The strains were grown out in BMMY media and tested for collagen and hydroxylation. The results are shown in Table 1 below.

### EXAMPLE 6

Example 2 was repeated following the same procedures and protocols with the following changes:
One DNA vector, MMV-78, containing both bovine P4HA and bovine P4HB sequences were inserted into the yeast. Both P4HA and P4HB contained their endogenous signal peptides and were driven by the Das1-Das2 bi-directional promoter. The DNA was digested by Kpnl and transformed into PP8 to generate PP3.

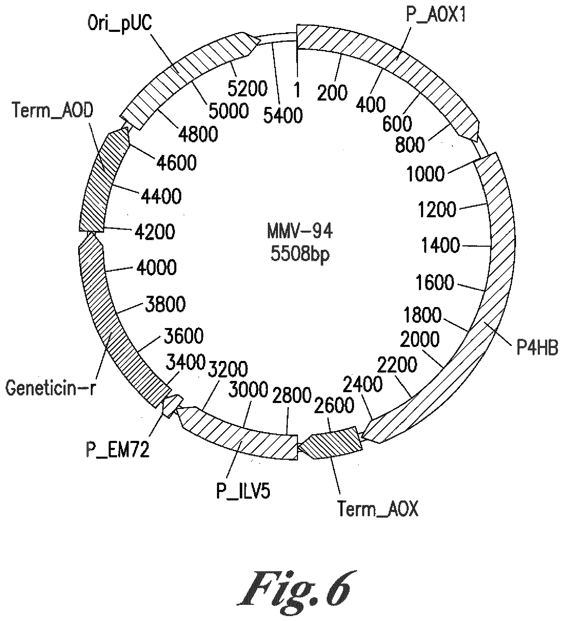

Another vector, MMV-94 (SEQ ID NO: 16) ("Sequence 14"), containing P4HB driven by pAOX1 promoter was used and was also inserted into the yeast. The endogenous signal peptide of P4HB was replaced by PHO1 signal peptide. The resulting strain was PP38. MMV94 was digested by Avr II and transformed into PP3 to generate PP38. The vector MMV94 is shown in FIG. 6. The strains were grown out in BMMY media and tested for collagen and hydroxylation. The results are shown in Table 1 below.

### EXAMPLE 7

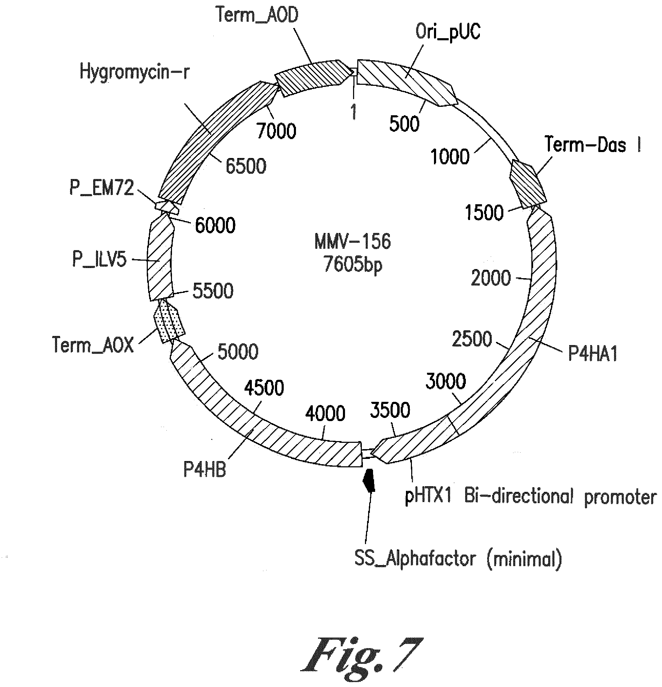

Example 4 was repeated following the same procedures and protocols with the following changes:
One DNA vector, MMV-156 (SEQ ID NO: 17) ("Sequence 15"), containing both bovine P4HA and bovine P4HB sequences were inserted into the yeast. The P4HA contained its endogenous signal peptides and P4HB signal sequence was replaced with Alpha-factor Pre (SEQ ID NO: 23) ("Sequence 21") sequence. Both genes were driven by the pHTX1 bi-directional promoter (SEQ ID NO: 26) ("Sequence 25"). MMV156 was digested by Barn HI and transformed into PP153 to generate PP154. The vector MMV156 is shown in FIG. 7. The strains were grown out in BMGY media and tested for collagen and hydroxylation. The results are shown in Table 1 below.

### EXAMPLE 8

Example 4 was repeated following the same procedures and protocols with the following changes: One DNA vector, MMV-156, containing both bovine P4HA and bovine P4HB sequences were inserted into the yeast. The P4HA contains its endogenous signal peptides and P4HB signal sequence was replaced with Alpha-factor Pre sequence. Both genes were driven by the pHTX1 bi-directional promoter. The DNA was digested by Swal and transformed into PP153 to generate PP154.

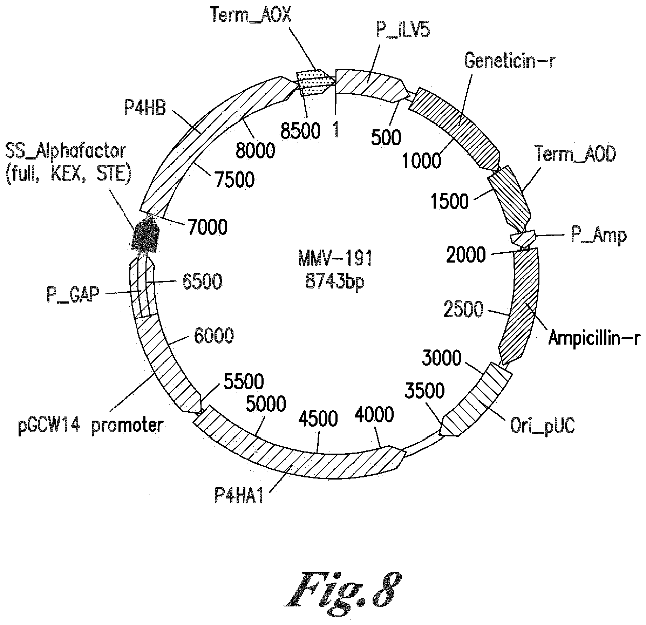

Another vector, MMV-191 (SEQ ID NO: 18) ("Sequence 16"), containing both P4HA and P4HB was also inserted into the yeast. The extra copy of P4HA contains its endogenous signal peptide and the signal sequence of the extra copy of P4HB was replaced with Alpha-factor Pre- Pro (SEQ ID NO: 24) ("Sequence 22") sequence. The extra copies of P4HA and P4HB were driven by the pGCW14-GAP1 bi-directional promoter (SEQ ID NO: 25) ("Sequence 23"). MMV191 was digested by Bam HI and transformed into PP154 to generate PP268. The vector MMV191 is shown in FIG. 8. The strains were grown out in BMGY media and tested for collagen and hydroxylation. The results are shown in Table 1 below.

### EXAMPLE 9

The methods and procedures of example 1 were utilized to create an all-in-one vector. The All-in-One vector contains DNA of collagen and associated promoter and terminator, the DNA for the enzymes that hydroxylate the collagen and associated promoters and terminators, the DNA for marker expression and associated promoter and terminator, the DNA for origin(s) of replication for bacteria and yeast, and the DNA(s) with homology to the yeast genome for integration. The All-in-one vector contains strategically placed unique restriction sites 5', 3', or within the above components. When any modification to collagen expression or other vector components is desired, the DNA for select components can easily be excised out with restriction enzymes and replaced with the user's chosen cloning method. The simplest version of the All-in-one vector MMV208 (SEQ ID NO: 19) ("Sequence 17") includes all of the above components except promoter(s) for hydroxylase enzymes. Vector MMV208 was made using the following components: AOX homology from MMV84 (SEQ ID NO: 20) ("Sequence 18"), Ribosomal homology from MMV150 (SEQ ID NO: 21) ("Sequence 19"), Bacterial and yeast origins of replication from MMV140 (SEQ ID NO: 22) ("Sequence 20"), Zeocin marker from MMV140, and Col3A1 from MMV129. Modified versions of P4HA and B and associated terminators were synthesized from Genscript eliminating the following restriction sites: AvrII, Notl, Pvul, Pmel, BamHI, Sacll, Swal, Xbal, Spel. The vector was transformed into strain PP1.

The strains were grown out in BMGY medium and tested for collagen and hydroxylation. The results are shown in Table 1 below.

Table 1 describes the amount of collagen produced in g/L as well as the percentage of hydroxylated collagen. The amount of collagen expressed was quantified by staining gels with Coomassie blue dye and comparing the result against a standard curve for collagen content. The amount of hydroxylated collagen was determined by comparing sample bands to a standard band after 1:25 pepsin treatment. Expression of hydroxylated collagen by *Pichia* is advantageous because hydroxylated collagen is stable in a high concentration of pepsin necessary to further process collagen polypeptides.

**Table 1**

| Example | Vector | Strain | Collagen (g/L) | Hydroxylated Collagen (%) |
|---|---|---|---|---|
| Wild type *Pichia pastoris* | none | PP1 | --- | --- |
| 1* | MMV-63 (SEQ ID NO: 11). Contains native bovine Type III collagen sequence (SEQ ID NO: 1) | PP28 | 0.05 | 0 |
| 2 | MMV-77 (SEQ ID NO: 12). Contains modified bovine collagen sequence (SEQ ID NO: 3) | PP8 | 0.1 | 0 |
| 3 | MMV-129 (SEQ ID NO: 13) contains modified bovine collagen sequence (SEQ ID NO: 3) and contains pCAT promoter (shown in SEQ ID NO: 9) to drive collagen expression. | PP123 | 0.5 | 0 |
| 4 | MMV-130 (SEQ ID NO: 14) containing codon-modified Type III bovine collagen sequence (SEQ ID NO: 3); pDF promoter (shown in SEQ ID NO: 8) used to drive collagen expression. AOX1 landing pad (SEQ ID NO: 10) facilitated site-specific integration of vector into *Pichia* genome. | PP153 | 1- 1.5 | 0 |
| 5* | MMV-77 (SEQ ID NO: 12). Contains modified bovine collagen sequence (SEQ ID NO: 3); and | PP3 | 0.1 | 15 |
| | MMV-78 | | | |
| | bovine P4HA (SEQ ID NO: 6) and P4HB (SEQ ID NO: 7) driven by Das1-Das2 bi-directional promoter (SEQ ID NO: 27) | | | |
| 6 | MMV-77 + MMV-78. MMV-94 contains P4HB driven by pAOX1 promoter, endogenous signal peptide of P4HB replaced by PHO1 signal peptide. | PP38 | 0.1 | 35 |
| 7 | MMV-130 containing Type III bovine collagen modified sequence (SEQ ID NO: 3), MMV156 | PP154 | 1- 1.5 | 15 |
| | bovine P4HA (endogenous signal peptide) and P4HB (alpha-factor pre sequence; SEQ ID NO: 23) | | | |
| 8 | MMV-130 + MMV-156. MMV-191 (SEQ ID NO: 18) contains bovine P4HA (endogenous signal peptide) and P4HB (alpha-factor pre-pro-sequence; SEQ ID NO: 24) sequences driven by the pGCW14-GAP1 bi-directional promoter (SEQ ID NO: 25). | PP268 | 1-1.5 | 40-50 |
| 9 | All-in-one vector | MMV-208 (SEQ ID NO: 19). | 0.5 - 1 | 15-20 |

The data in Examples 1 and 2 show that codon-modification of the Type III bovine collagen sequence doubled the amount collagen expressed by *Pichia.* Comparison of the data from Examples 2 and 3 shows that expression of Type III bovine collagen is further increased by a factor of 5 by driving transcription of the Type III collagen coding sequence with the pCAT promoter. Comparison of data from Examples 2 and 4 show that bovine Type III collagen expression is increased ten to fifteen-fold by driving transcription of the Type III collagen coding sequence with the pDF promoter and providing an AOX1 landing pad to facilitate integration of the vector into genomic DNA of *Pichia.* Comparison of data from Examples 2, and 5 and 6 shows that transformation of *Pichia* with coding sequences for proline hydroxylase (P4HA + P4HB) produced hydroxylated collagen and that the amount of hydroxylated collagen could be increased by further regulating expression of the proline hydroxylase. Examples 7-9 show that collagen expression can be boosted by five to fifteen-fold and that the amount of hydroxylate collagen increased either by introducing two vectors or by an all-in-one vector approach where both collagen and hydroxylase sequences are encoded by the same vector.

### EXAMPLE 10

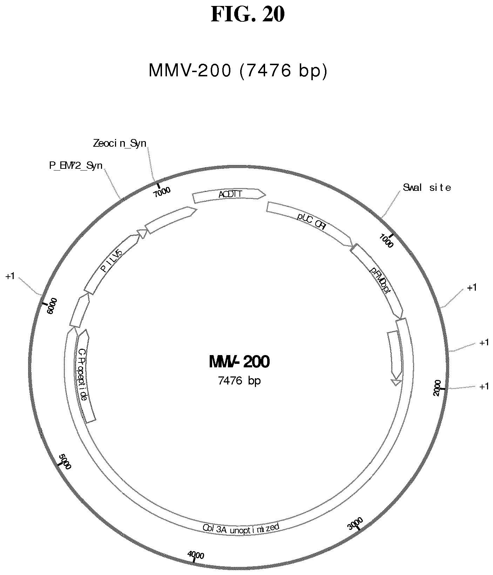

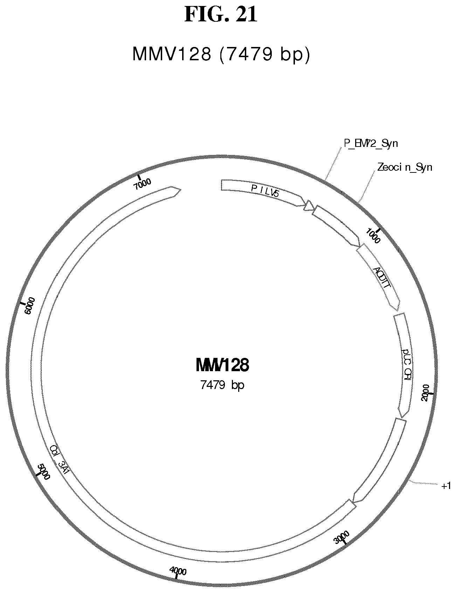

The methods and procedures of example 1 were utilized to create chimeric Col3A1 vectors. The vector MMV132 was modified to include the DNA of chimeric collagen and associated promoter PDF and terminator AOX1TT, the DNA for marker expression and associated promoter and terminator, the DNA for origin(s) of replication for bacteria and yeast, and the DNA(s) with homology to the yeast genome for integration. Vector MMV63 was the source DNA for the unmodified Col3A1 domains. Vector MMV128 (Figure 21) was the source DNA for the modified Col3A1 domains. The total length of Col3A1 polypeptide is 1465 amino acids (aa). Plasmids were designed to incorporate native Bovine DNA sequences (unmodified) and *Pichia pastoris* codon modified DNA sequences. Plasmids were designed such that transitions between modified and unmodified sequences of Col3A1 were at aa 710, 1,200, and 1,331. These methods were used to create plasmids MMV193, MMV194, MMV195, MMV197, MMV198, and MMV199. The resulting plasmid vectors are shown in Table 2 below with the fully optimized plasmid MMV130 and fully unoptimized plasmid MMV200 (FIG. 20) for comparison.

**Table 2**

| Split Point | First Half | Second Half | Plasmids |
|---|---|---|---|
| None | Optimized | Optimized | MMV130 |
| 710 | Optimized | Unoptimized | MMV193 |
| 1220 | Optimized | Unoptimized | MMV194 |
| 1331 | Optimized | Unoptimized | MMV195 |
| 710 | Unoptimized | Optimized | MMV197 |
| 1220 | Unoptimized | Optimized | MMV198 |
| 1331 | Unoptimized | Optimized | MMV199 |
| None | Unoptimized | Unoptimized | MMV200 |

### EXAMPLE 11

Example 2 was repeated following the same procedures and protocols with the following changes:
PP1 and PP97 were obtained. PP97 was a strain where two protease genes (PEP4 and PRB1) were knocked out from the host strain. The DNA MMV194, MMV195, MMV130 and MMV200 sequences including different combinations of modified and unmodified bovine collagen sequence DNA for *Pichia* expression were inserted into the yeast. A pDF promoter was used to drive the expression of collagen sequence. A YPD plate containing Zeocin at 500 ug/ml was used to select successful transformants. Restriction digestion was done using Swa I to linearize DNA for integration, 3-5ug of cut DNA was transformed for vectors except for MMV130 which was digested with Pme1 and 200 ng of DNA was transformed. The resulting strains are shown in Table 3 below.

**Table 3**

| Parent Strain | Split Point | First Half | Second Half | Plasmids | Strain |
|---|---|---|---|---|---|
| PP1 | None | Optimized | Optimized | MMV130 | PP153 |
| PP1 | 1220 | Optimized | Unoptimized | MMV194 | PP205 |
| PP1 | 1331 | Optimized | Unoptimized | MMV195 | PP206 |
| PP1 | None | Unoptimized | Unoptimized | MMV200 | PP328 |
| PP97 | None | Optimized | Optimized | MMV130 | PP333 |
| PP97 | 1220 | Optimized | Unoptimized | MMV194 | PP266 |
| PP97 | 1331 | Optimized | Unoptimized | MMV195 | PP267 |
| PP97 | None | Unoptimized | Unoptimized | MMV200 | PP334 |

### EXAMPLE 12

Example 7 was repeated following the same procedures and protocols with the following changes:
One DNA vector, MMV-156, containing both bovine P4HA and bovine P4HB sequences was inserted into the yeast. The P4HA contains its endogenous signal peptides and the P4HB signal sequence was replaced with Alpha-factor Pre sequence. Both genes were driven by the pHTX1 bi-directional promoter. The DNA was digested by BamH1 and transformed. See Table 4 for strain and transformation information.

**Table 4**

| Parent Strain | Split Point | First Half | Second Half | Plasmids | Strain |
|---|---|---|---|---|---|
| PP153 | None | Optimized | Optimized | MMV156 | PP154 |
| PP205 | 1220 | Optimized | Unoptimized | MMV156 | PP275 |
| PP206 | 1331 | Optimized | Unoptimized | MMV156 | PP276 |
| PP328 | None | Unoptimized | Unoptimized | MMV156 | PP332 |
| PP333 | None | Optimized | Optimized | MMV156 | PP349 |
| PP266 | 1220 | Optimized | Unoptimized | MMV156 | PP273 |
| PP267 | 1331 | Optimized | Unoptimized | MMV156 | PP274 |
| PP334 | None | Unoptimized | Unoptimized | MMV156 | PP344 |

### EXAMPLE 13

Example 8 was repeated following the same procedures and protocols with the following changes:
The lysis buffer is made with 50 mM Na₂PO₄, 1mM EDTA, 5% glycerol, and the pH adjusted to 7.4 with acetic acid. Another vector, MMV-191, containing both P4HA and P4HB, was also inserted into the yeast. The extra copy of P4HA contains its endogenous signal peptide and the signal sequence of the extra copy of P4HB was replaced with Alpha-factor Pre-Pro sequence. The extra copies of P4HA and P4HB were driven by the pGCW14-GAP1 bi-directional promoter. The DNA was digested by Barn HI and transformed. See Table 5 for transformation and new strain information. The strains were grown out in BMGY media and tested for collagen.

**Table 5**

| Parent Strain | Split Point | First Half | Second Half | Plasmids | Strain | Collagen (g/L) |
|---|---|---|---|---|---|---|
| PP154 | None | Optimized | Optimized | MMV191 | PP268 | 0.12 |
| PP275 | 1220 | Optimized | Unoptimized | MMV191 | PP329 | 0.16 |
| PP276 | 1331 | Optimized | Unoptimized | MMV191 | PP330 | 0.16 |
| PP332 | None | Unoptimized | Unoptimized | MMV191 | PP347 | 0.12 |
| PP349 | None | Optimized | Optimized | MMV191 | PP407 | 0.09 |
| PP273 | 1220 | Optimized | Unoptimized | MMV191 | PP292 | 0.27 |
| PP274 | 1331 | Optimized | Unoptimized | MMV191 | PP293 | 0.22 |
| PP344 | None | Unoptimized | Unoptimized | MMV191 | PP346 | 0.12 |

### EXAMPLE 14

Example 2 was repeated following the same procedures and protocols with the following changes:
One DNA vector, MMV-78, containing both bovine P4HA and bovine P4HB sequences was inserted into the yeast. P4HA and P4HB are driven by the Das1-Das2 bi-directional promoter. The DNA was digested by Kpn I and transformed into PP8 to generate PP3 which contains the collagen sequence of SEQ ID NO: 3 ("Sequence 2"). Another vector, MMV-94 (SEQ ID NO: 16) ("Sequence 14"), containing P4HB driven by pAOX1 promoter was used and was also inserted into the yeast. The endogenous signal peptide of P4HB was replaced by PHO1 signal peptide. The resulting strain was PP38.

A 24-deepwell plate was filled with 2 ml YPD in each well and single colonies of strain PP38 were inoculated. The colonies were grown in YPD for 24 hours with shaking at 900 rpm. The cells were spun down at 3,000 rpm for 5 minutes and the supernatant was removed. For methanol-free induction, the supernatant was replaced with 2 mL BMGY (1%) and grown for another 48 hours. For methanol induction, methanol was added to a final concentration 0.5% and the cells grown for 24 hours. Methanol was added again and the cells grown for another 24 hours. At the end of induction, 1 ml of sample was removed for analysis.

The samples were tested for collagen using SDS-PAGE and Coomassie staining described in Example 1. The band for the methanol-free induction sample was darker than the band for the methanol induced sample, showing the methanol-free induction sample had a higher concentration of expressed collagen.

Terms such as "optimized" or "optimize" as used herein include values or characteristics realized by careful selection of features of chimeric DNA constructs or other critical process variables and do not imply use of a known results-effective variable.

Terminology used herein is for the purpose of describing particular embodiments only and is not intended to be limiting of the invention.

The headings (such as "Background" and "Summary") and sub-headings used herein are intended only for general organization of topics within the present invention, and are not intended to limit the disclosure of the present invention or any aspect thereof. In particular, subject matter disclosed in the "Background" may include novel technology and may not constitute a recitation of prior art. Subject matter disclosed in the "Summary" is not an exhaustive or complete disclosure of the entire scope of the technology or any embodiments thereof. Classification or discussion of a material within a section of this specification as having a particular utility is made for convenience, and no inference should be drawn that the material must necessarily or solely function in accordance with its classification herein when it is used in any given composition.

As used herein, the singular forms "a", "an" and "the" are intended to include the plural forms as well, unless the context clearly indicates otherwise.

As used herein, the term "and/or" includes any and all combinations of one or more of the associated listed items and may be abbreviated as "/".

Links are disabled by insertion of a space or underlined space before "www" and may be reactivated by removal of the space.

As used herein in the specification and claims, including as used in the examples and unless otherwise expressly specified, all numbers may be read as if prefaced by the word "substantially", "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is+/- 0.1% of the stated value (or range of values),+/- 1% of the stated value (or range of values),+/- 2% of the stated value (or range of values), +/- 5% of the stated value (or range of values),+/- 10% of the stated value (or range of values),+/- 15% of the stated value (or range of values),+/- 20% of the stated value (or range of values), etc. Any numerical range recited herein is intended to include all sub-ranges subsumed therein.

As used herein, the words "preferred" and "preferably" refer to embodiments of the technology that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the technology.

As referred to herein, all compositional percentages are by weight of the total composition, unless otherwise specified. As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present invention that do not contain those elements or features.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

The citation of references herein does not constitute an admission that those references are prior art or have any relevance to the patentability of the technology disclosed herein. Any discussion of the content of references cited is intended merely to provide a general summary of assertions made by the authors of the references, and does not constitute an admission as to the accuracy of the content of such references.

## Claims

1. A chimeric bovine collagen DNA sequence comprising a combination of a section of SEQ ID NO: 1 which has been optimized for expression in *Pichia pastoris* and a section of SEQ ID NO: 1 that is unoptimized, wherein the section of SEQ ID NO: 1 which has been optimized for expression in *Pichia pastoris* comprises 60 to 90% of the total length of the chimeric bovine collagen DNA sequence, and the section of SEQ ID NO: 1 that is unoptimized comprises 10 to 40% of the total length of the chimeric collagen DNA sequence, and wherein the section of SEQ ID NO: 1 which has been optimized for expression in *Pichia pastoris* encodes the amino acid sequence at the N-terminus of a bovine collagen molecule and the section of SEQ ID NO: 1 that is unoptimized encodes the amino acid sequence at the C-terminus of a bovine collagen molecule.

2. The chimeric bovine collagen DNA sequence of claim 1, wherein the chimeric bovine collagen DNA sequence is linked to a polynucleotide sequence encoding P4HA1, P4HB, or both.

3. The chimeric bovine collagen DNA sequence of claim 1, wherein the section of SEQ ID NO: 1 which has been optimized for expression in *Pichia pastoris* comprises 60 to 80% of the total length of the chimeric bovine collagen DNA sequence.

4. A strain of collagen-producing yeast comprising a vector, wherein the vector comprises the chimeric bovine collagen DNA sequence of claim 1.

5. The strain of collagen-producing yeast of claim 4, further comprising a polynucleotide sequence encoding P4HA1, P4HB, or both.

6. A method for producing hydroxylated collagen comprising growing the strain of collagen-producing yeast of claim 5 in a medium for 24 hours to 72 hours.

7. The strain of collagen-producing yeast of claim 4, wherein the strain of collagen-producing yeast is a strain of Pichia.

8. The method of claim 6, wherein the medium is selected from the group consisting of buffered glycerol complex media, buffered methanol complex media, and yeast extract peptone dextrose.

9. The method of claim 6, wherein the period of time is 24 hours, 48 hours, or 72 hours.

10. The method of claim 6, wherein the vector further comprises a promoter selected from the group consisting of a pTHX1 constitutive bi-directional promoter and a pGCW14-pGAP1 constitutive bi-directional promoter.

11. The method of claim 6, wherein the vector further comprises at least one selection marker selected from the group consisting of DNA encoding an antibiotic resistance and DNA encoding an auxotrophic marker.

## Patentansprüche

1. Chimäre DNA-Sequenz von Rinderkollagen, umfassend eine Kombination eines Abschnitts von SEQ ID Nr. 1, der zur Expression in *Pichia pastoris* optimiert worden ist, und eines Abschnitts von SEQ ID Nr. 1, der nicht optimiert ist, wobei der Abschnitt von SEQ ID Nr. 1, der zur Expression in *Pichia pastoris* optimiert worden ist, 60 bis 90 ö der Gesamtlänge der chimären DNA-Sequenz von Rinderkollagen umfasst und der Abschnitt von SEQ ID Nr. 1, der nicht optimiert ist, 10 bis 40 ö der Gesamtlänge der chimären DNA-Sequenz von Kollagen umfasst, und wobei der Abschnitt von SEQ ID Nr. 1, der zur Expression in *Pichia pastoris* optimiert worden ist, die Aminosäuresequenz am N-Terminus eines Rinderkollagenmoleküls codiert und der Abschnitt von SEQ ID Nr. 1, der nicht optimiert ist, die Aminosäuresequenz am C-Terminus eines Rinderkollagenmoleküls codiert.

2. Chimäre DNA-Sequenz von Rinderkollagen nach Anspruch 1, wobei die chimäre DNA-Sequenz von Rinderkollagen mit einer Polynucleotidsequenz, die P4HA1, P4HB oder beide codiert, verknüpft ist.

3. Chimäre DNA-Sequenz von Rinderkollagen nach Anspruch 1, wobei der Abschnitt von SEQ ID Nr. 1, der zur Expression in *Pichia pastoris* optimiert worden ist, 60 bis 80 ö der Gesamtlänge der chimären DNA-Sequenz von Rinderkollagen umfasst.

4. Stamm von Kollagen produzierender Hefe, umfassend einen Vektor, wobei der Vektor die chimäre DNA-Sequenz von Rinderkollagen nach Anspruch 1 umfasst.

5. Stamm von Kollagen produzierender Hefe nach Anspruch 4, weiterhin umfassend eine Polynucleotidsequenz, die P4HA1, P4HB oder beide codiert.

6. Verfahren zur Herstellung von hydroxyliertem Kollagen, umfassend das Züchten des Stamms von Kollagen produzierender Hefe nach Anspruch 5 in einem Medium über 24 Stunden bis 72 Stunden.

7. Stamm von Kollagen produzierender Hefe nach Anspruch 4, wobei der Stamm von Kollagen produzierender Hefe ein Pichia-Stamm ist.

8. Verfahren nach Anspruch 6, wobei das Medium ausgewählt ist aus der Gruppe bestehend aus gepufferten Glycerol-Komplexmedien, gepufferten Methanol-Komplexmedien und Hefeextrakt-Pepton-Dextrose.

9. Verfahren nach Anspruch 6, wobei die Zeitspanne 24 Stunden, 48 Stunden oder 72 Stunden beträgt.

10. Verfahren nach Anspruch 6, wobei der Vektor weiterhin einen Promotor umfasst, der ausgewählt ist aus der Gruppe bestehend aus einem konstitutiven bidirektionalen Promotor, pTHX1, und einem konstitutiven bidirektionalen Promotor, pGCW14-pGAP1.

11. Verfahren nach Anspruch 6, wobei der Vektor weiterhin mindestens einen Selektionsmarker umfasst, der ausgewählt ist aus der Gruppe bestehend aus DNA, die eine Antibiotikaresistenz codiert, und DNA, die einen auxotrophen Marker codiert.

## Revendications

1. Séquence d'ADN de collagène bovin chimérique comprenant une combinaison d'une section de SEQ ID n° 1 qui a été optimisée pour l'expression dans *Pichia pastoris* et une section de SEQ ID n° 1 qui est non optimisée, la section de SEQ ID n° 1 qui a été optimisée pour l'expression dans *Pichia pastoris* comprenant 60 à 90 % de la longueur totale de la séquence d'ADN de collagène bovin chimérique et la section de SEQ ID n° 1 qui est non optimisée comprenant 10 à 40 % de la séquence d'ADN de collagène chimérique et la section de SEQ ID n° 1 qui a été optimisée pour l'expression dans *Pichia pastoris* code pour la séquence d'acides aminés à l'extrémité N-terminale d'une molécule de collagène bovin et la section de SEQ ID n° 1 qui est non optimisée code pour la séquence d'acides aminés à l'extrémité C-terminale d'une molécule de collagène bovin.

2. Séquence d'ADN de collagène bovin chimérique selon la revendication 1, dans laquelle la séquence d'ADN de collagène bovin chimérique est liée à une séquence polynucléotidique codant pour P4HA1, P4HB ou les deux.

3. Séquence d'ADN de collagène bovin chimérique selon la revendication 1, dans laquelle la section de SEQ ID n° 1 qui a été optimisée pour l'expression dans *Pichia pastoris* comprend 60 à 80 % de la longueur totale de la séquence d'ADN de collagène bovin chimérique.

4. Souche de levure produisant du collagène comprenant un vecteur, dans laquelle le vecteur comprend la séquence d'ADN de collagène bovin chimérique selon la revendication 1.

5. Souche de levure produisant du collagène selon la revendication 4, comprenant en outre une séquence polynucléotidique codant pour P4HA1, P4HB ou les deux.

6. Procédé de production de collagène hydroxylé comprenant la croissance de la souche de levure produisant du collagène selon la revendication 5 dans un milieu durant 24 heures à 72 heures.

7. Souche de levure produisant du collagène selon la revendication 4, dans laquelle la souche de levure produisant du collagène est une souche de Pichia.

8. Procédé selon la revendication 6, dans lequel le milieu est choisi dans le groupe constitué par des milieux complexes de glycérol tamponné, des milieux complexes de méthanol tamponné et du peptone dextrose d'extrait de levure.

9. Procédé selon la revendication 6, dans lequel la période de temps est de 24 heures, de 48 heures ou de 72 heures.

10. Procédé selon la revendication 6, dans lequel le vecteur comprend en outre un promoteur choisi dans le groupe constitué par un promoteur bi-directionnel constitutif pTHX1 et un promoteur bi-directionnel constitutif pGCW14-pGAP1.

11. Procédé selon la revendication 6, dans lequel le vecteur comprend en outre au moins un marqueur de sélection choisi dans le groupe constitué par l'ADN codant pour une résistance antibiotique et l'ADN codant pour un marqueur auxotrophe.
